(19) Europäisches Patentamt

European Patent Office

Office européen des brevets

(11) **EP 3 818 996 A1**

(12) **EUROPÄISCHE PATENTANMELDUNG**

(43) Veröffentlichungstag:
**12.05.2021 Patentblatt 2021/19**

(51) Int Cl.:
***A61M 1/10*** *(2006.01)*     ***A61M 1/12*** *(2006.01)*

(21) Anmeldenummer: **19208405.1**

(22) Anmeldetag: **11.11.2019**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Berlin Heart GmbH**
**12247 Berlin (DE)**

(72) Erfinder:
 • **STEINGRÄBER, Dr.-Ing. Robert**
  **14055 Berlin (DE)**
 • **KIESNER, Dr.-Ing. Matthias**
  **14050 Berlin (DE)**

 • **REIPRICH, Dr. rer. nat. Alexander**
  **17279 Lychen (DE)**
 • **WOODARD, Dr. John C.**
  **Turramurra, New South Wales 2074 (AU)**

(74) Vertreter: **Pfenning, Meinig & Partner mbB**
**Patent- und Rechtsanwälte**
**Joachimsthaler Straße 10-12**
**10719 Berlin (DE)**

Bemerkungen:
Die Patentansprüche 16 - 21 gelten als fallen gelassen, da die entsprechenden Anspruchsgebühren nicht entrichtet wurden (R. 45(3) EPÜ).

(54) **PUMPENSYSTEM, STEUEREINHEIT UND VERFAHREN ZUM BETREIBEN EINES PUMPENSYSTEMS**

(57)     Die Anmeldung betrifft ein Pumpensystem (1), umfassend eine Membranfluidpumpe (2), die mittels einer Einlasskanüle (9) und einer Auslasskanüle (11) mit einem Herzen und/oder mindestens einem Blutgefäß fluidisch verbindbar und zum Erzeugen eines pulsatilen Fluidflusses zur Unterstützung einer Herzaktivität des Herzens eingerichtet ist, eine Arbeitsdruckquelle (3), die mittels einer Druckleitung (15) mit der Membranfluidpumpe (2) verbunden und zum Bereitstellen eines Arbeitsdrucks zum Antreiben der Membranfluidpumpe (2) eingerichtet ist, eine Steuereinheit (4), eingerichtet zum Regeln des Arbeitsdrucks, einen ersten Flusssensor (21), eingerichtet zum Erfassen eines ersten Kanülenflusssignals, das einem Einlassfluss in der Einlasskanüle (9) oder einem Auslassfluss in der Auslasskanüle (11) entspricht, einen Arbeitsdrucksensor (20), eingerichtet zum Erfassen eines Arbeitsdrucksignals, das dem Arbeitsdruck in der Druckleitung (15) entspricht, wobei der pulsatile Fluidfluss mehrere aufeinanderfolgende Pumpzyklen umfasst, die Herzaktivität mehrere aufeinanderfolgende Herzzyklen umfasst und jeder der Pumpzyklen eine Füllungsphase und eine Entleerungsphase umfasst. Die Steuereinheit (4) ist ferner dazu eingerichtet, einen Zeitversatz zwischen einem ersten Zeitpunkt, der während eines ersten Pumpzyklus auftritt, und einem zweiten Zeitpunkt, der während eines ersten Herzzyklus auftritt, zu bestimmen, basierend auf dem Zeitversatz und/oder dem ersten Kanülenflusssignal und/oder dem Arbeitsdrucksignal einen hämodynamischen Parametersatz, umfassend einen oder mehrere hämodynamische Parameter, zu bestimmen und basierend auf einer Regelvorgabe als Vorgabe für den hämodynamischen Parametersatz und/oder für den Zeitversatz eine Zeitvorgabe als Vorgabe für einen Beginn und/oder eine Dauer der Füllungsphase und/oder der Entleerungsphase des ersten Pumpzyklus und/oder mindestens eines zweiten Pumpzyklus, der zeitlich nach dem ersten Pumpzyklus auftritt, derart zu bestimmen, dass die Regelvorgabe erreicht wird. Die Anmeldung betrifft ferner eine Steuereinheit (4) für ein Pumpensystem (1) sowie ein Verfahren zum Betreiben eines Pumpensystems (1).

EP 3 818 996 A1

Fig. 1

**Beschreibung**

Die vorliegende Anmeldung betrifft ein Pumpensystem, umfassend eine Membranfluidpumpe, die zum Erzeugen eines pulsatilen Fluidflusses zur Unterstützung einer Herzaktivität eingerichtet ist, eine Steuereinheit für ein solches Pumpensystem sowie ein Verfahren zum Betrieb eines solchen Pumpensystems.

Pumpensysteme zur Herzunterstützung, also zur Unterstützung einer Herzaktivität, sind aus dem Stand der Technik bekannt und werden im Folgenden auch als auch als VAD (ventricular assist device) bezeichnet. In solchen Pumpensystemen können zum Pumpen von Blut verschiedene Arten von Blutpumpen zum Einsatz kommen, etwa Membranfluidpumpen oder Rotationsfluidpumpen. Membranfluidpumpen zeichnen sich dadurch aus, dass damit ein pulsatiler Fluidfluss, ähnlich der natürlichen Herzaktivität, erzeugbar ist.

Bei der Verwendung eines VADs können physiologische Ereignisse oder Änderungen beim Patienten in vielen Fällen nur unzureichend erfasst werden, womit auch eine angemessene Reaktion auf solche Ereignisse oder Änderungen, etwa durch Nachregeln von Betriebsparametern der Membranfluidpumpe, erschwert wird.

Beispielsweise soll bei Patienten, deren Herzaktivität sich während der Behandlung mit einem VAD verbessert, eine Entwöhnung (auch Weaning) durch schrittweise Verringerung des Beitrags des VADs zum Gesamtblutfluss im Kreislauf des Patienten erzielt werden, was nur durch Messung und Auswertung veränderlicher hämodynamischer Parameter erreicht werden kann. Dies ist oft nicht möglich oder mit invasiven Diagnoseverfahren verbunden.

Als weiteres Beispiel kann bei Patienten mit Fontan-Zirkulation eine Verschlechterung der Herzaktivität bis hin zum Herzversagen auftreten (Failing Fontan), bei dessen Behandlung mittels VAD es von großer Bedeutung ist, auf Druckänderungen in den Hohlvenen gegensteuernd zu reagieren, um einerseits zu hohe Drücke und deren negative Folgen (etwa Leber- oder sonstige Organschädigungen), andererseits durch Unterdruck verursachtes Kollabieren der Hohlvenen zu vermeiden. Auch hierfür fehlen geeignete Pumpensysteme.

Bei einigen akuten Erkrankungen des Herzmuskels wie einem Herzinfarkt hilft eine starke Entlastung, die Langzeitschäden zu reduzieren. Die Narbengröße bleibt durch die Entlastung klein. Es gibt aber auch Patienten, die am Ende einer Therapie mit Herz-Kreislauf-Unterstützung vom System entwöhnt werden sollen. Bei ihnen soll der Herzmuskel wieder belastet werden. Gleichzeitig soll das Unterstützungssystem aber noch einen Mindestblutfluss sicherstellen.

Allgemein fehlt es also an Pumpensystemen, die entsprechend der tatsächlich vorliegenden Gegebenheiten, insbesondere Momentan- und/oder Langzeitwerten hämodynamischer Parameter, zum Bereitstellen eines bedarfsgerechten Blutflusses regelbar sind, gleichzeitig aber physiologische Randbedingungen, die die Sicherheit und Gesundheit des Patienten sicherstellen, berücksichtigen (zu diesen Randbedingungen können etwa eine Gewährleisten einer regelmäßigen Öffnung der Aorten- oder Pulmonalklappe oder ein Vermeiden eines Ansaugens der Herzwand oder Kollabierens der Hohlvenen).

Eine Aufgabe der vorliegenden Anmeldung besteht dementsprechend darin, ein Pumpensystem vorzuschlagen, welches die genannten Nachteile vermeidet oder verringert. Weiterhin sollen eine Steuereinheit für ein solches Pumpensystem sowie ein Verfahren zum Betreiben eines solchen Pumpensystems vorgeschlagen werden.

Die Aufgabe wird gelöst durch ein Pumpensystem nach Anspruch 1, eine Steuereinheit nach Anspruch 20 und ein Verfahren zum Betreiben eines Pumpensystems nach Anspruch 21. Vorteilhafte Ausgestaltungen und Weiterentwicklungen ergeben sich mit den Merkmalen der Unteransprüche.

Das vorgeschlagene Pumpensystem umfasst

eine Membranfluidpumpe, die mittels einer Einlasskanüle und einer Auslasskanüle mit einem Herzen und/oder mindestens einem Blutgefäß fluidisch verbindbar und zum Erzeugen eines pulsatilen Fluidflusses zur Unterstützung einer Herzaktivität des Herzens eingerichtet ist,
eine Arbeitsdruckquelle, die mittels einer Druckleitung mit der Membranfluidpumpe verbunden und zum Bereitstellen eines Arbeitsdrucks zum Antreiben der Membranfluidpumpe eingerichtet ist,
eine Steuereinheit, eingerichtet zum Regeln des Arbeitsdrucks,
einen ersten Flusssensor, eingerichtet zum Erfassen eines ersten Kanülenflusssignals, das einem Einlassfluss in der Einlasskanüle oder einem Auslassfluss in der Auslasskanüle entspricht,
einen Arbeitsdrucksensor, eingerichtet zum Erfassen eines Arbeitsdrucksignals, das dem Arbeitsdruck in der Druckleitung entspricht,
wobei der pulsatile Fluidfluss mehrere aufeinanderfolgende Pumpzyklen umfasst, die Herzaktivität mehrere aufeinanderfolgende Herzzyklen umfasst und jeder der Pumpzyklen eine Füllungsphase und eine Entleerungsphase umfasst.

Als Membranfluidpumpe wird hier eine Vorrichtung bezeichnet, in deren Inneren ein Hohlraum ausgebildet ist, der durch eine auslenkbare Membran in eine Pumpfluidkammer und eine Arbeitsfluidkammer geteilt wird.

Die Pumpfluidkammer weist einen mit der Einlasskanüle verbindbaren oder verbundenen Fluideinlass sowie einen mit der Auslasskanüle verbindbaren oder verbundenen Fluidauslass auf. Durch eine Membranbewegung, insbesondere durch Auslenken der Membran in einander entgegengesetzte Richtungen, kann die Größe der Pumpfluidkammer variiert und damit ein Unter- oder Überdruck erzeugt werden, durch den ein Fluid, insbesondere Blut, in die Pumpfluidkammer einströmen oder aus dieser ausströmen kann.

Die Membranfluidpumpe und/oder die Einlass- und/oder Auslasskanüle kann Ventile umfassen, die beispielsweise am Fluideinlass und/oder am Fluidauslass angeordnet sein und so steuerbar sein können, dass das Fluid im Betrieb durch den Einlass einströmen und durch den Auslass ausströmen kann. Die Ventile können auch Einwegventile sein, so dass auch ohne eine aktive Steuerung erreicht werden kann, dass das Fluid nur durch den Einlass einströmen und/oder nur durch den Auslass ausströmen kann.

Durch wiederholtes Auslenken der Membran zum aufeinanderfolgenden Vergrößern und Verkleinern der Pumpfluidkammer wird der pulsatile Fluidfluss erzeugt, wobei jeder Pumpzyklus die durch das Einströmen von Fluid in die Pumpfluidkammer definierte Füllungsphase (auch Füllphase genannt) und die durch das Ausströmen von Fluid aus der Pumpfluidkammer definierte Entleerungsphase (nicht notwendigerweise in dieser Reihenfolge) umfasst. Zwischen den aufeinanderfolgenden Füllungs- und Entleerungsphasen können Pausen der Membranbewegung vorgesehen sein.

Die Arbeitsfluidkammer ist mit einem Arbeitsfluid füllbar und mittels der Druckleitung mit der Arbeitsdruckquelle so verbunden, dass mittels der Arbeitsdruckquelle ein Überdruck und/oder ein Unterdruck in der Arbeitsfluidkammer zum Auslenken der Membran erzeugt werden kann.

Die Arbeitsdruckquelle kann beispielsweise eine Kolbenpumpe, umfassend einen in einem Arbeitsraum bewegbaren Arbeitskolben, eine andere Art von Pumpe oder ein Pneumatikschaltkreis, umfassend einen Kompressor, einen Überdruckbehälter und einen Unterdruckbehälter, sein. Das Arbeitsfluid kann vorzugsweise Luft, aber auch ein anderes Gas oder auch eine Flüssigkeit sein.

Das mittels des Arbeitsdrucksensors erfasste Arbeitsdrucksignal kann von der Steuereinheit als Überwachungs- und/oder Rückkopplungssignal zum Regeln des Arbeitsdrucks und somit zum Regeln des pulsatilen Fluidflusses verwendet werden.

Sämtliche vorstehend und nachfolgend genannten Drücke innerhalb des Pumpensystems können insbesondere Relativdrücke, also insbesondere relativ zum Umgebungsdruck gemessene Drücke oder auch relativ zu einem Thorax-Druck gemessene Drücke, sein.

Zur Verwendung des Pumpensystems zur Herzunterstützung kann die Membranfluidpumpe und/oder die Einlasskanüle und/oder die Auslasskanüle wenigstens teilweise implantierbar sein. Ferner kann die Einlasskanüle beispielsweise mit einem Ventrikel und/oder einem Vorhof und/oder einer Hohlvene und/oder einer Pulmonalvene, die Auslasskanüle mit einer Aorta und/oder einer Pulmonalarterie verbindbar sein.

Der erste Flusssensor kann zum Erfassen des ersten Kanülenflusssignals in oder an der Einlasskanüle oder der Auslasskanüle oder dem Fluideinlass oder dem Fluidauslass angeordnet sein. Der erste Flusssensor kann beispielsweise ein Ultraschallflusssensor, ein Hall-Effekt-Sensor oder eine andere Art von Flusssensor sein.

Jeder Herzzyklus weist typischerweise eine Füllungsphase (Diastole, gekennzeichnet durch Entspannung des Herzmuskels) und eine Entleerungsphase (Systole, gekennzeichnet durch Kontraktion des Herzmuskels) auf (nicht notwendigerweise in dieser Reihenfolge). Die Herzaktivität ist durch eine Reihe wichtiger Parameter gekennzeichnet, von denen einige im Folgenden erläutert werden.

Die Dauer eines Herzzyklus wird als Periodendauer der Herzaktivität bezeichnet, deren Kehrwert als Pumprate. Unter Vorlast wird die Kraft verstanden, die am Ende der Diastole der Füllung der Ventrikel entgegenwirkt. Häufig wird die Vorlast durch den enddiastolischen Druck, also die am gefüllten Ventrikel anliegende Druckdifferenz (in Bezug auf den Umgebungsdruck oder, vorzugsweise, den Thorax-Druck), oder das enddiastolische Volumen, also das Ventrikel-Füllvolumen am Ende der Diastole angenähert. Die Nachlast ist die Kraft, die der Kontraktion des Herzmuskels in der Systole entgegengesetzt ist, und kann über den arteriellen Widerstand bzw. den arteriellen Blutdruck abgeschätzt werden. Als Kontraktilität bezeichnet man die Fähigkeit des Herzmuskels, durch Kontraktion Kraft auszuüben bzw. Druck aufzubauen.

Es ist vorgesehen, dass die Steuereinheit ferner dazu eingerichtet ist,

einen Zeitversatz zwischen einem ersten Zeitpunkt, der während eines ersten Pumpzyklus auftritt, und einem zweiten Zeitpunkt, der während eines ersten Herzzyklus auftritt, zu bestimmen,
basierend auf dem Zeitversatz und/oder dem ersten Kanülenflusssignal und/oder dem Arbeitsdrucksignal einen hämodynamischen Parametersatz, umfassend einen oder mehrere hämodynamische Parameter, zu bestimmen und basierend auf einer Regelvorgabe als Vorgabe für den hämodynamischen Parametersatz und/oder für den Zeitversatz eine Zeitvorgabe als Vorgabe für einen Beginn und/oder eine Dauer der Füllungsphase und/oder der Entleerungsphase des ersten Pumpzyklus und/oder mindestens eines zweiten Pumpzyklus, der zeitlich nach dem ersten Pumpzyklus auftritt, derart zu bestimmen, dass die Regelvorgabe erreicht wird.

Der erste Zeitpunkt kann anhand von Merkmalen, insbesondere wiederholt und/oder periodisch auftretenden Merkmalen eines Zeitverlaufs einer Messgröße und/oder eines Pumpenparameters definiert sein, beispielsweise eines Drucksignals und/oder eines Flusssignals, insbesondere des Arbeitsdrucksignals und/oder des ersten Kanülenflusssignals. Der zweite Zeitpunkt kann anhand von Merkmalen, insbesondere wiederholt und/oder periodisch auftretenden Merkmalen in Abhängigkeit des Zeitversatzes eines Zeitverlaufs einer Messgröße und/oder eines hämodynamischen Parameters definiert sein, beispielsweise eines Drucksignals und/oder eines Flusssignals, etwa eines Kanülendrucksignals und/oder des ersten Kanülenflusssignals, oder auch beispielsweise eines EKG-Signals.

Der erste Zeitpunkt kann beispielsweise ein Beginn oder ein Ende der Füllungsphase oder der Entleerungsphase des ersten Pumpzyklus oder ein sonstiger Zeitpunkt während des ersten Pumpzyklus sein. Entsprechend kann der zweite Zeitpunkt beispielsweise ein Beginn oder ein Ende der Füllungsphase oder der Entleerungsphase des ersten Pumpzyklus oder ein sonstiger Zeitpunkt während des ersten Herzzyklus sein.

Der Zeitversatz kann anhand von Merkmalen, insbesondere wiederholt und/oder periodisch auftretenden Merkmalen und/oder in Abhängigkeit des Zeitversatzes veränderlichen Merkmalen eines Zeitverlaufs einer Messgröße und/oder eines Pumpenparameters und/oder eines hämodynamischen Parameters definiert sein, beispielsweise eines Drucksignals und/oder eines Flusssignals, insbesondere des Arbeitsdrucksignals und/oder des ersten Kanülenflusssignals. Der Zeitversatz kann dabei auch implizit definiert und/oder implizit bestimmbar, d. h. ohne explizites Bestimmen des ersten und zweiten Zeitpunkts bestimmbar sein.

Die Steuereinheit kann etwa dazu eingerichtet sein, den Zeitversatz basierend auf einem Sensorsignal oder mehreren Sensorsignalen zu bestimmen. Beispielsweise kann die Steuereinheit dazu eingerichtet sein, den Zeitversatz basierend auf dem ersten Kanülenflusssignal als Sensorsignal zu bestimmen. Dies wird dadurch ermöglicht, dass das erste Kanülenflusssignal ein Gesamtflusssignal ist, das also nicht nur Informationen über den Beitrag der Membranfluidpumpe zum Gesamtfluss, sondern auch Informationen über den Beitrag der Herzaktivität zum Gesamtfluss enthält. Alternativ oder zusätzlich kann die Steuereinheit dazu eingerichtet sein, den Zeitversatz basierend auf einem oder mehreren weiteren Sensorsignalen, beispielsweise dem Arbeitsdrucksignal und/oder einem elektrokardiographischen Signal (EKG-Signal) und/oder einem Kanülendrucksignal zu bestimmen.

Der mindestens eine zweite Pumpzyklus kann ein unmittelbar auf den ersten Pumpzyklus folgender Pumpzyklus (oder mehrere solche Pumpzyklen) oder ein erst um einen oder mehrere Pumpzyklen später auftretender Pumpzyklus (oder mehrere solche Pumpzyklen), beispielsweise auch ein erst erheblich später (beispielsweise Minuten oder Stunden später) auftretender Pumpzyklus (oder mehrere solche Pumpzyklen) sein.

Der Zeitversatz stellt eine wichtige Kenngröße des Betriebs des Pumpensystems dar, deren Bestimmen grundlegend ist für ein Erkennen physiologischer Ereignisse oder Änderungen beim Patienten, die als Änderungen des Zeitversatzes und/oder basierend auf dem Zeitversatz bestimmbarer weiterer Größen, beispielsweise der hämodynamischen Parameter des hämodynamischen Parametersatzes, erfassbar sind.

Dadurch wird eine angemessene Reaktion auf solche Ereignisse oder Änderungen, insbesondere durch Nachregeln von Betriebsparametern der Membranfluidpumpe, insbesondere der Zeitvorgabe, möglich. Entsprechend der Wahl des zweiten Pumpzyklus kann die Zeitvorgabe dabei ein schnelles/unmittelbar wirkendes Nachregeln oder ein graduelles/längerfristig wirkendes (dann wiederum aufgrund der kontinuierlichen Erfassung des Zeitversatzes korrigierbares) Nachregeln ermöglichen. Dazu kann die Steuereinheit auch dazu eingerichtet sein, neben der genannten Zeitvorgabe, die sich auf den ersten und/oder zweiten Pumpzyklus bezieht, auch weitere Zeitvorgaben, die sich auf weitere Pumpzyklen beziehen, zu bestimmen.

Dem Bestimmen des Zeitversatzes und dem darauf basierenden Bestimmen des hämodynamischen Parametersatzes und der Zeitvorgabe liegt die Erkenntnis zugrunde, dass der Zeitversatz die Eigenschaft der Membranfluidpumpe als zusätzliches, grundsätzlich unabhängig von der Herzaktivität füllbares und entleerbares Reservoir im Kreislauf wiederspiegelt, dessen in Bezug auf die Herzaktivität steuerbare Füllungs- und Entleerungsphasen zahlreiche Vorteile mit sich bringen. Insbesondere kann eine Kenntnis und/oder Variation des Zeitversatzes zum Messen und/oder Steuern wichtiger Größen ausgenutzt werden. Diese Möglichkeiten fehlen beispielsweise bei einer durch Drehzahlvariation pulsatil betriebenen Rotationsfluidpumpe aufgrund des fehlenden zusätzlichen Reservoirs.

Da somit die Membranfluidpumpe selbst als Messgerät verwendet wird, kann der hämodynamische Parametersatz nichtinvasiv im Betrieb des Pumpensystems bestimmt und die Zeitvorgabe entsprechend bestimmt werden.

Zusammenwirkend haben das Bestimmen des Zeitversatzes, das darauf basierende Bestimmen des hämodynamischen Parametersatzes und das Bestimmen der Zeitvorgabe also den Vorteil, dass das Pumpensystem entsprechend der tatsächlich vorliegenden Gegebenheiten, insbesondere Momentan- oder Langzeitwerten hämodynamischer Parameter, zum Bereitstellen eines bedarfsgerechten Blutflusses regelbar ist und gleichzeitig physiologische Randbedingungen, die die Sicherheit und Gesundheit des Patienten sicherstellen, durch die Regelvorgabe und/oder die Zeitvorgabe berücksichtigt werden können.

Die Regelvorgabe kann einen bestimmten Wert oder Wertebereich eines oder mehrerer hämodynamischer Parameter des hämodynamischen Parametersatzes und/oder des Zeitversatzes umfassen, kann aber auch andere Arten von Vorgaben, beispielsweise eine bloße Richtungsvorgabe, etwa ein Erhöhen, Absenken oder Konstanthalten eines oder mehrerer hämodynamischer Parameter des hämodynamischen Parametersatzes und/oder des Zeitversatzes umfassen.

Bestimmen des hämodynamischen Parametersatzes bzw. Bestimmen eines oder mehrerer hämodynamischer Parameter kann auch ein Bestimmen von Näherungswerten und/oder Größen, die sich wenigstens in bestimmten Wertebereichen ähnlich wie der oder die jeweiligen hämodynamischen Parameter verhalten, bedeuten (wie etwa im oben genannten Beispiel der Annäherung der Vorlast durch das enddiastolische Volumen etc.). Ähnlich verhalten kann hierbei beispielsweise eine wenigstens näherungsweise Proportionalität oder ein anderer wenigstens bereichsweise umkehrbarer funktionaler Zusammenhang sein.

Bestimmen der Zeitvorgabe derart, dass die Regelvorgabe erreicht wird, bedeutet nicht notwendigerweise, dass die Regelvorgabe im Betrieb tatsächlich erreicht wird. Bestimmen der Zeitvorgabe auf diese Art kann auch deuten, dass die Zeitvorgabe so bestimmt wird, dass die Regelvorgabe unter einer oder mehrerer Annahmen voraussichtlich erreicht wird, beispielsweise eine oder mehrere der folgenden Annahmen: eine Annahme, gemäß der die Zeitvorgabe durch die Steuereinheit umgesetzt wird; eine Annahme, gemäß der sich die hämodynamischen Parameter nicht so ändern, dass die Regelvorgabe nicht mehr erreicht werden kann; eine Annahme, gemäß der die Regelvorgabe nicht zum Einstellen von Stellgrößen außerhalb jeweiliger Stellgrößenbegrenzungen führt; eine Annahme, gemäß der sich die hämodynamischen Parameter so ändern, dass die Regelvorgabe mittels einer Zeitvorgabe, die innerhalb einer unteren und einer oberen Grenze liegt, erreicht werden kann.

Die Steuereinheit kann dazu eingerichtet sein, den Arbeitsdruck basierend auf der Zeitvorgabe so zu regeln, dass die Regelvorgabe erreicht wird.

Damit wird eine automatische, insbesondere durch Rückkopplung des hämodynamischen Parametersatzes und/oder des Zeitversatzes erzielte, Regelung des Pumpensystems, also eine automatische Reaktion auf physiologische Ereignisse oder Änderungen ermöglicht, welche den Vorteil hat, zumindest in bestimmten Situationen kein Einschreiten des Benutzers oder von medizinischem Personal zu erfordern.

Regeln des Arbeitsdrucks derart, dass die Regelvorgabe erreicht wird, bedeutet dabei nicht notwendigerweise, dass die Regelvorgabe im Betrieb tatsächlich erreicht wird. Regeln des Arbeitsdrucks auf diese Art bedeutet vielmehr, dass der Arbeitsdruck derart geregelt wird, dass die Regelvorgabe unter der Annahme voraussichtlich erreicht wird, dass sich die hämodynamischen Parameter nicht so ändern, dass die Regelvorgabe nicht mehr erreicht werden kann.

Das Pumpensystem kann ferner eine Anzeigeeinheit aufweisen, wobei die Anzeigeeinheit zum Anzeigen einer basierend auf der Zeitvorgabe bestimmten vorgeschlagenen Einstellungsänderung und/oder zum Anzeigen mindestens eines hämodynamischen Parameters des hämodynamischen Parametersatzes eingerichtet ist.

Alternativ oder zusätzlich zu der automatischen Regelung des Pumpensystems kann auf diese Weise die benötigte Information für eine manuelle Regelung bzw. ein manuelles Einschreiten ermöglicht werden, was den Vorteil hat, dass eine Einschätzung und Beurteilung der Situation etwa durch medizinisches Personal als Grundlage der Regelung ermöglicht wird.

Die Steuereinheit kann dazu eingerichtet sein, einen oder mehrere hämodynamische Parameter des hämodynamischen Parametersatzes anzuzeigen und/oder auf einem Datenträger zu speichern. Ein Aufzeichnen eines oder mehrerer hämodynamische Parameter des hämodynamischen Parametersatzes erlaubt ein Auswerten zu einem späteren Zeitpunkt, beispielsweise durch medizinisches Personal. Ein Anzeigen eines oder mehrerer hämodynamischer Parameter des hämodynamischen Parametersatzes erlaubt beispielsweise eine diagnostische Verwendung durch medizinisches Personal.

Der hämodynamische Parametersatz kann als hämodynamische Parameter eine Pumprate des Herzens und/oder die Vorlast des Herzens und/oder einen enddiastolischen Druck und/oder ein enddiastolisches Volumen und/oder die Nachlast des Herzens und/oder die Kontraktilität des Herzens und/oder einen Ventrikeldruck und/oder ein Ventrikelfüllvolumen und/oder einen Vorhofdruck und/oder ein Vorhoffüllvolumen und/oder einen Arteriendruck und/oder einen Venendruck umfassen.

Wie bereits oben diskutiert, sind die genannten Parameter Beispiele wichtiger hämodynamischer Parameter bzw. Kenngrößen der Herzaktivität, womit ihr Bestimmen im Betrieb des Pumpensystems sowohl aus diagnostischer als auch aus regeltechnischer Sicht vorteilhaft ist.

Konkrete Beispiele des Bestimmens der hämodynamischen Parameter des hämodynamischen Parametersatzes basierend auf dem Zeitversatz und/oder dem ersten Kanülenflusssignal und/oder dem Arbeitsdrucksignal werden weiter unten beschrieben.

Der hämodynamische Parametersatz kann als hämodynamische Parameter ein Pumpenfüllvolumen und/oder einen mittleren Blutfluss, bestimmt als über mehrere Pumpzyklen gemittelter mittlerer Blutfluss durch die Membranfluidpumpe, und/oder einen einer relativen Lage der Membranfluidpumpe in Bezug auf das Herz entsprechenden hydrostatischen Druck umfassen

Die Steuereinheit kann dazu eingerichtet sein, den Arbeitsdruck so zu regeln, dass die Membranfluidpumpe in der Füllungsphase jedes Pumpzyklus vollständig gefüllt und/oder in der Entleerungsphase jedes Pumpzyklus vollständig entleert wird.

Dabei wird unter vollständigem Füllen bzw. Entleeren eine vollständige Bewegung der Membran in jeweilige Endlagen verstanden. Am Ende der Entleerungsphase kann ein Restpumpfluidvolumen, am Ende der Füllungsphase ein Restarbeitsfluidvolumen vorhanden sein. Durch das vollständige Füllen bzw. Entleeren kann eine Bildung von Thromben und/oder eine Unterversorgung des Patienten vermieden werden.

Es kann vorgesehen sein, dass die Zeitvorgabe eine Vorgabe für den Beginn und/oder die Dauer der Füllungsphase und/oder der Entleerungsphase des ersten und/oder zweiten Pumpzyklus ist,

wobei der hämodynamische Parametersatz den mittleren Blutfluss, bestimmt als über mehrere Pumpzyklen gemittelter mittlerer Blutfluss durch die Membranfluidpumpe, umfasst,

wobei die Regelvorgabe eine Vorgabe für den hämodynamischen Parametersatz, insbesondere umfassend eine Vorgabe für den mittleren Blutfluss, ist und

wobei die Steuereinheit weiterhin dazu eingerichtet ist, als Regelvorgabe ein Erhöhen, Absenken oder Konstanthalten des mittleren Blutflusses basierend auf einem Zeitverlauf des hämodynamischen Parametersatzes zu bestimmen.

Durch Regeln des mittleren Blutflusses gemäß der so bestimmten Regelvorgabe kann ein Grad der Herzunterstützung, also sowohl der insgesamt erzeugte Volumenstrom des pulsatilen Fluidflusses, als auch der Anteil des pulsatilen Blutflusses am Gesamtblutfluss eingestellt werden, womit besonders gut auf physiologische Änderungen und Ereignisse reagiert werden kann. Beispielsweise eignet sich das Pumpensystem in diesem Fall besonders gut zur Entwöhnung wie oben beschrieben.

Das Pumpensystem kann weiterhin einen Beschleunigungssensor und/oder Drehratensensor aufweisen, eingerichtet zum Erfassen eines einer Patientenaktivität entsprechenden Beschleunigungssignals, wobei die Steuereinheit dazu eingerichtet ist, die Regelvorgabe basierend auf dem Beschleunigungssignal zu bestimmen, insbesondere so, dass bei einem Ansteigen der Patientenaktivität der mittlere Blutfluss erhöht wird.

Da der Sauerstoff- und somit Blutflussbedarf von der Patientenaktivität abhängt, kann auf diese Weise auf das physiologische Ereignis veränderten Sauerstoffbedarfs besonders gut reagiert werden.

Es kann ferner vorgesehen sein, dass der hämodynamische Parametersatz neben einer Kontraktilität des Herzens eine Vorlast des Herzens und/oder eine Nachlast des Herzens umfasst und die Steuereinheit dazu eingerichtet ist,

als Regelvorgabe ein Absenken des mittleren Blutflusses zu bestimmen, wenn der Zeitverlauf des hämodynamischen Parametersatzes ein Ansteigen der Kontraktilität ohne Ansteigen der Vorlast und/oder ohne Ansteigen der Nachlast umfasst und/oder wenn der Zeitverlauf des hämodynamischen Parametersatzes ein Absinken der Vorlast ohne Absinken der Kontraktilität umfasst, und/oder

als Regelvorgabe ein Konstanthalten des mittleren Blutflusses zu bestimmen, wenn der Zeitverlauf des hämodynamischen Parametersatzes ein Ansteigen der Kontraktilität und ein Ansteigen der Vorlast und/oder der Nachlast umfasst, und/oder

als Regelvorgabe ein Erhöhen des mittleren Blutflusses zu bestimmen, wenn der Zeitverlauf des hämodynamischen Parametersatzes ein Ansteigen der Vorlast ohne Ansteigen der Kontraktilität umfasst und/oder wenn der Zeitverlauf des hämodynamischen Parametersatzes ein Ansteigen der Nachlast ohne Absinken der Vorlast und ohne Ansteigen des Ventrikelhöchstdrucks umfasst und/oder wenn der Zeitverlauf des hämodynamischen Parametersatzes ein Absinken der Kontraktilität ohne Absinken der Vorlast und/oder ohne Absinken der Nachlast umfasst.

Die Kontraktilität, die Nachlast und die Vorlast sind - wie bereits oben beschrieben - wichtige Kenngrößen der Herzaktivität und spielen auch eine zentrale Rolle in der Beschreibung des Frank-Starling-Mechanismus, der einen wichtigen autonomen Regelkreis der Hämodynamik beschreibt (Zusammenhang zwischen Fülldruck/Füllvolumen und Herzzeitvolumen, also gefördertem Volumenstrom pro Zeiteinheit). Indem die Steuereinheit zum Bestimmen der Regelvorgabe wie vorstehend beschrieben eingerichtet ist, wird eine physiologisch vorteilhafte Steuerung des mittleren Blutflusses und somit des Grades der Herzunterstützung erreicht.

Die Steuereinheit kann dazu eingerichtet sein, den Zeitversatz gemäß der Gleichung

$$T = \frac{\tau}{2\pi} \arg\left( \left[ \sqrt{\frac{1}{t_1 - t_0} \int_{t_0}^{t_1} Q(t)^2 \, dt} + i \int_{t_S}^{t_E} \left( \frac{Q(t) - \langle Q \rangle}{\sigma_Q} \right)^3 \, dt \right] e^{i\alpha} \right)$$

zu bestimmen, wobei T der Zeitversatz, $\tau$ eine Periodendauer des pulsatilen Fluidflusses, Q(t) ein Zeitverlauf des ersten Kanülenflusssignals, t ein Zeitparameter, $t_S$ der Zeitpunkt, zu dem Q(t) zum letzten Mal vor dem Erreichen eines Maxi-

malflusses $Q_{max}$ weniger als 20 % von $Q_{max}$ beträgt, $t_E$ der Zeitpunkt, zu dem Q(t) zum letzten Mal seit Erreichen von $Q_{max}$ weniger als 20 % von $Q_{max}$ beträgt, <Q> der Mittelwert von Q(t) im Zeitintervall von $t_S$ bis $t_E$, $\sigma_Q$ die Standardabweichung von Q(t) im Zeitintervall von $t_S$ bis $t_E$, $t_0$ der Zeitpunkt in der Mitte der Entleerungsphase des Pumpzyklus, $t_1$ der Zeitpunkt in der Mitte der Entleerungsphase des folgenden Pumpzyklus (d. h. $t_1$ - $t_0$ ist die Periodendauer), i die imaginäre Einheit, $\alpha$ ein vorgegebener Winkeloffset ist. Diese einfach umsetzbare Möglichkeit des Bestimmens des Zeitversatzes wird weiter unten näher erläutert.

Das Pumpensystem kann weiterhin umfassen

einen EKG-Sensor, eingerichtet zum Erfassen eines EKG-Signals, das einer elektrischen Aktivität des Herzens entspricht,
und/oder einen ersten Kanülendrucksensor, eingerichtet zum Erfassen eines ersten Kanülendrucksignals, das einem Einlassdruck in der Einlasskanüle oder einem Auslassdruck in der Auslasskanüle entspricht,
wobei die Steuereinheit dazu eingerichtet ist, den Zeitversatz und/oder den hämodynamischen Parametersatz basierend auf dem EKG-Signal und/oder dem ersten Kanülendrucksignal zu bestimmen.

Umfasst das Pumpensystem einen EKG-Sensor, so kann insbesondere der Zeitpunkt des Beginns der Entleerungsphase eines Herzzyklus (etwa des ersten Herzzyklus), also der Ventrikelsystole, und somit ein darauf basierender Zeitversatz und/oder hämodynamischer Parameter besonders zuverlässig und genau bestimmt werden.

Zu diesem Zweck kann die Steuereinheit dazu eingerichtet sein, einen periodischen Anteil des EKG-Signals zu erkennen. Beispielsweise kann die Steuereinheit dazu eingerichtet sein, einen QRS-Komplex als Anteil des EKG-Signals zu erkennen. Die Steuereinheit kann auch zum Erkennen einer T-Welle und/oder einer P-Welle als Anteil des EKG-Signals eingerichtet sein. Daraus ergibt sich zusätzliche Information über den Zeitverlauf des Herzzyklus bzw. der Herzaktivität, woraus wiederum Zeitversätze und/oder hämodynamische Parameter besonders gut bestimmt werden können.

Der pulsatile Fluidfluss kann eine Messphase umfassen, wobei die Steuereinheit dazu eingerichtet ist, für die Dauer der Messphase den Arbeitsdruck auf einen geringen Arbeitsdruckwert so einzustellen, dass eine hohe Empfindlichkeit des ersten Kanülenflusssignals für durch den ersten Herzzyklus verursachte Druckänderungen erreicht wird.

Durch Vorsehen einer Messphase kann das unabhängige Reservoir der Membranfluidpumpe besonders gut zur Bestimmung des Zeitversatzes und/oder hämodynamischer Parameter ausgenutzt werden.

Es kann weiterhin vorgesehen sein, dass die Zeitvorgabe eine Vorgabe für den Beginn und/oder die Dauer der Füllungsphase und/oder der Entleerungsphase des ersten und/oder zweiten Pumpzyklus ist
und dass die Regelvorgabe eine Vorgabe für den Zeitversatz derart ist, dass eine vorgegebene relative Phasenlage des pulsatilen Fluidflusses in Bezug auf die Herzaktivität erreicht wird, insbesondere ein Gleichpuls oder ein Gegenpuls oder ein um ein vorgegebenes Verzögerungsintervall verzögerter Puls.

Als relative Phasenlage wird hier eine zeitliche Beziehung zwischen dem pulsatilen Fluidfluss und der Herzaktivität bezeichnet, d. h. ein Zeitversatz (nämlich das Verzögerungsintervall) zwischen einem Zeitpunkt, durch den der pulsatile Fluidfluss bzw. der Arbeitsdruck charakterisiert ist und einem Zeitpunkt, durch den die Herzaktivität charakterisiert ist. Der relative Phasenversatz kann beispielsweise nur zwischen den Füllphasen von Pumpzyklus und Herzzyklus, nur zwischen den Entleerungsphasen von Pumpzyklus und Herzzyklus, zwischen sowohl den Füllphasen und den Entleerungsphasen von Pumpzyklus und Herzzyklus, oder auch zwischen Füllphasen des Pumpzyklus und Entleerungsphasen des Herzzyklus oder zwischen Entleerungsphasen des Pumpzyklus und Füllphasen des Herzzyklus bestehen.

Die Zeitpunkte, durch die für die Definition der relativen Phasenlage der pulsatile Fluidfluss und die Herzaktivität charakterisiert sind, können Beginn und/oder Ende von Füll- und/oder Entleerungsphasen, Zeitpunkte maximalen und/oder minimalen Flusses oder andere zeitliche Merkmale des pulsatilen Fluidflusses und der Herzaktivität sein.

Als Gleichpuls wird eine relative Phasenlage bezeichnet, bei der ein Zeitversatz zwischen einem Zeitpunkt der Füllphase des Pumpzyklus und einem Zeitpunkt der Füllphase des Herzzyklus wenigstens näherungsweise Null ist und/oder bei der ein Zeitversatz zwischen einem Zeitpunkt der Entleerungsphase des Pumpzyklus und einem Zeitpunkt der Entleerungsphase des Herzzyklus wenigstens näherungsweise Null ist, die Füllung und/oder Entleerung der Pumpe und des Herzens also teilweise oder vollständig gleichzeitg auftreten.

Als Gegenpuls wird eine relative Phasenlage bezeichnet, bei der ein Zeitversatz zwischen einem Zeitpunkt der Füllphase des Pumpzyklus und einem Zeitpunkt der Entleerungsphase des Herzzyklus wenigstens näherungsweise Null ist und/oder bei der ein Zeitversatz zwischen einem Zeitpunkt der Entleerungsphase des Pumpzyklus und einem Zeitpunkt der Füllphase des Herzzyklus wenigstens näherungsweise Null ist, die Füllung der Pumpe also wenigstens teilweise gleichzeitig mit der Entleerung des Herzens und/oder die Entleerung der Pumpe teilweise gleichzeitig mit der Füllung des Herzens auftritt.

Gleichpuls und Gegenpuls können also auch als Spezialfälle des vorgegebenen Verzögerungsintervalls definiert werden, bei denen das vorgegebene Verzögerungsintervall wenigstens näherungsweise Null ist.

Die relative Phasenlage kann zeitlich wenigstens für ein gewisses Zeitintervall konstant sein, was dann der Fall ist, wenn die Periodendauern bzw. Pumpraten des pulsatilen Fluidflusses und der Herzaktivität übereinstimmen. Die relative Phasenlage kann auch zeitlich veränderlich sein, was sich aus einer anfänglich festgelegten Phasenlage bei unterschiedlichen Periodendauern bzw. Pumpraten des pulsatilen Fluidflusses und der Herzaktivität ergibt.

Durch das Einstellen der vorgegebenen relativen Phasenlage kann also eine vorteilhafte konstante oder veränderlich Beziehung zwischen Herzzyklus und Pumpzyklus erzielt werden, weshalb das Einstellen der vorgegebenen relativen Phasenlage im Folgenden auch kurz als Synchronisation bezeichnet wird, obwohl Herzzyklus und Pumpzyklus hierbei nicht tatsächlich synchron sein müssen. Das Einstellen der vorgegebenen relativen Phasenlage zwischen den Füll- und Entleerungszeiten von Ventrikeln und Membranfluidpumpe kann auch einen wichtigen Beitrag zur gezielten Be- und Entlastung des Herzmuskels liefern. Wenn die Membranfluidpumpe sich während der Diastole des Herzens füllt, leistet der Herzmuskel besonders wenig Arbeit, verbraucht wenig Sauerstoff und kann sich regenerieren. Wenn die Blutpumpe sich während der Systole des Herzens füllt, wird der Herzmuskel wieder stärker belastet. Durch die fortdauernde Unterstützung mit einer vorgegebenen Mindestrate sowie einer Entleerungsdauer in einem zulässigen Bereich besteht jedoch nicht die Gefahr von zu langen Stagnationsphasen in der Membranfluidpumpe oder die Gefahr einer Unterversorgung des Patienten.

Die Steuereinheit kann zum Erzielen der Synchronisation mittels eines schnellen Verfahrens eingerichtet sein, bei dem die Synchronisation noch innerhalb des ersten Herzzyklus basierend auf dem bestimmten Zeitversatz erzielt wird. Um dies zu erreichen, kann die Steuereinheit dazu eingerichtet sein, beim Erfassen einer Änderung des ersten Kanülenflusssignals und/oder des ersten Drucksignals und/oder beim Erkennen einer periodischen Komponente des EKG-Signals sofort die Zeitvorgabe als das vorgegebene Verzögerungsintervall zu bestimmen und den Arbeitsdruck gemäß der Zeitvorgabe so zu regeln, dass die relative Phasenlage noch im ersten Herzzyklus erreicht wird. Das schnelle Verfahren kann auch über mehrere Pumpzyklen in (kleineren) Einzelschritten angewandt werden. Wenn der Zeitversatz schnell, aber über mehrere Pumpzyklen angepasst wird, wird das Risiko einer ungleichmäßigen Versorgung stark vermindert.

Die Steuereinheit kann auch zum Erzielen der Synchronisation auf andere Weise, beispielsweise mittels eines im Vergleich mit dem vorstehend beschriebenen Verfahren langsameren Ratenvariationsverfahren erzielt werden. Dazu kann vorgesehen sein, dass die Steuereinheit dazu eingerichtet ist,

eine Periodendauer des pulsatilen Fluidflusses über mehrere Pumpzyklen zu variieren,
eine durch das Variieren der Periodendauer verursachte Änderung eines pro Pumpzyklus bestimmten Maximalwertes und/oder Minimalwertes und/oder Mittelwertes und/oder Momentanwertes und/oder eines sonstigen Zeitverlaufsmerkmals (etwa einer Schiefe) des ersten Kanülenflusssignals zu erfassen und
die Zeitvorgabe so zu bestimmen, dass der pro Pumpzyklus bestimmte Maximalwert und/oder Minimalwert und/oder Mittelwert und/oder Momentanwert des ersten Kanülenflusssignals maximiert oder minimiert wird.

Entspricht bei diesem Ratenvariationsverfahren etwa das erste Kanülenflusssignal dem Einlassfluss und wird die Zeitvorgabe so bestimmt, dass der Maximalwert des ersten Kanülenflusssignals maximiert wird, so wird als relative Phasenlage ein Gegenpuls erzielt; wird dagegen der Maximalwert des ersten Kanülenflusssignals minimiert, so wird als relative Phasenlage ein Gleichpuls erzielt. Das Ratenvariationsverfahren erlaubt eine besonders schonende Synchronisation mit geringen Nebenwirkungen.

Es kann vorgesehen sein,

dass die Regelvorgabe eine Vorgabe für den Zeitversatz ist und
dass die Steuereinheit dazu eingerichtet ist, bei einem Erfassen einer Abweichung des durch die Steuereinheit bestimmten Zeitversatzes von der Regelvorgabe die Periodendauer des pulsatilen Fluidflusses über mehrere Pumpzyklen in eine erste Richtung zu ändern, bis ein Vorzeichenwechsel einer Ableitung eines pro Pumpzyklus bestimmten Maximalwertes und/oder Minimalwertes und/oder Mittelwertes und/oder Momentanwertes und/oder eines sonstigen Zeitverlaufsmerkmals des ersten Kanülenflusssignals erfasst wird,
und ferner dazu eingerichtet ist, beim Erfassen des Vorzeichenwechsels die Periodendauer des pulsatilen Fluidflusses in die der ersten Richtung entgegengesetzte Richtung zu ändern.

Auf diese Weise (insbesondere durch regelmäßiges Wiederholen des Verfahrens) kann eine besonders stabile und genaue Synchronisation erzielt werden, wobei die Steuereinheit auf physiologische Änderungen gut reagieren kann.

Die Steuereinheit kann dazu eingerichtet sein, basierend auf einem Zeitverlauf des ersten Kanülenflusssignals einen Druck und/oder eine Druckänderung und/oder eine Druckänderungsrate in einem mit der Membranfluidpumpe verbundenen Ventrikel und/oder Vorhof und/oder Blutgefäß zu bestimmen.

Dies wird dadurch ermöglicht, dass die für das erste Kanülenflusssignal relevanten Flüsse von Drücken in den damit verbundenen Ventrikeln bzw. Vorhöfen bzw. Blutgefäßen beeinflusst werden. Damit können wichtige Druckgrößen bzw. deren Verläufe auch ohne entsprechend platzierte Drucksensoren abgeschätzt werden oder, falls solche Drucksensoren vorhanden sind, zusätzliche Information gewonnen werden. Beispielsweise kann anhand des Zeitverlaufs des Einlassflusses eine Erhöhung des Drucks in einem Ventrikel oder einem Blutgefäß, beispielsweise auch in einer oder zweier mit der Membranfluidpumpe verbundener Hohlvenen erkannt und/oder der Druck in den Hohlvenen geschätzt werden, (letzteres kann beispielsweise bei Failing-Fontan-Patienten wichtig sein).

Weiterhin kann das Herzunterstützungssystem zum Bestimmen mindestens eines Lageparameters eines Patienten eingerichtet sein. Die Steuereinheit kann zum Berechnen eines durch eine Flüssigkeitssäule in der Einlasskanüle und/oder der Auslasskanüle verursachten zusätzlichen Drucks zum Bestimmen des Lageparameters eingerichtet sein. Das Herzunterstützungssystem kann einen zum Bestimmen des Lageparameters eingerichteten Sensor, beispielsweise einen gyroskopischen Sensor, aufweisen.

Das Bestimmen eines Drucks und/oder einer Druckänderung und/oder einer Druckänderungsrate in einem mit der Membranfluidpumpe verbundenen Ventrikel und/oder Vorhof und/oder Blutgefäß und kann durch eine Positionierung der Membranfluidpumpe in der Nähe des bzw. auf der Höhe des sogenannten (venösen) hydrodynamischen Indifferenzpunkts (HIP) begünstigt werden, wozu das Bestimmen des mindestens einen Lageparameters nützlich sein kann. Bei niedrigen Füllungsdrücken hängt der Fluss besonders stark vom Druck am proximalen Einlasskanülenende ab. Durch die Positionierung der Membranfluidpumpe auf der Höhe des HIP hat die Patientenlage keinen Einfluss auf das Bestimmen des Drucks und/oder der Druckänderung und/oder der Druckänderungsrate. Falls eine Positionierung der Membranfluidpumpe auf der Höhe des HIP nicht möglich ist, kann somit beim Bestimmen des Drucks etc. auch der bestimmte Lageparameter des Patienten einbezogen werden.

Die Steuereinheit kann dazu eingerichtet sein, beim Erkennen einer Änderung eines Drucks in einem mit der Membranfluidpumpe verbundenen Blutgefäß die Periodendauer des pulsatilen Blutflusses so zu ändern, dass die erkannte Änderung des Drucks teilweise oder vollständig kompensiert wird.

Die Steuereinheit kann dazu eingerichtet sein, den Zeitversatz durch Variieren der Zeitvorgabe über mehrere Pumpzyklen so zu variieren, dass der Druck und/oder die Druckänderung und/oder die Druckänderungsrate in dem mit der Membranfluidpumpe verbundenen Ventrikel und/oder Vorhof und/oder Blutgefäß basierend auf einer durch das Variieren des Zeitversatzes verursachten Änderung des Zeitverlaufs des ersten Kanülenflusssignals bestimmbar ist.

Auf diese Weise kann wiederum das zusätzliche Reservoir, das durch die Membranfluidpumpe im Blutkreislauf bereitgestellt wird, besonders gut für eine Messung ausgenutzt werden.

Der hämodynamische Parametersatz kann einen Momentanfüllstand der Membranfluidpumpe, bestimmt basierend auf einem Integral des ersten Kanülenflusssignals, umfassen,
wobei die Steuereinheit dazu eingerichtet ist, den Momentanfüllstand mit einem zeitlich veränderlichen Momentansollfüllstand der Membranfluidpumpe zu vergleichen und, wenn der Momentanfüllstand den Momentansollfüllstand um mindestens einen vorgegebenen Wert unterschreitet, den Arbeitsdruck so zu regeln, dass eine Pumprate des pulsatilen Fluidflusses und/oder ein Saugdruck, mit dem die Membranfluidpumpe gefüllt wird, verringert wird.

Der Momentansollfüllstand ist typischerweise eine zeitlich veränderliche Funktion, die einen Sollfüllstand der Membranfluidpumpe zu jedem Zeitpunkt vorgibt. Unterschreitet der Momentanfüllstand den Momentansollfüllstand um mindestens den vorgegebenen Wert, so ist dies ein Indiz für ein physiologisches Ereignis, dass die Einlassflussrate verringert. Ein solches physiologisches Ereignis kann etwa ein Ansaugen einer Herzwand oder ein Kollabieren einer Hohlvene sein (letzteres ist besonders bei Failing-Fontan-Patienten von Bedeutung); das Verringern der Pumprate bzw. des Saugdrucks wirkt einem solchen Ereignis entgegen.

Ein möglicher Nachteil dieser Methode ist, dass die Pumprate bzw. der Saugdruck im Laufe der Zeit zu stark verringert werden kann.

Um dies zu verhindern, kann die Steuereinheit ferner dazu eingerichtet sein, die Pumprate des pulsatilen Fluidflusses und/oder den Saugdruck über mehrere Pumpzyklen zu erhöhen, bis der Momentanfüllstand den Momentansollfüllstand um mindestens den vorgegebenen Wert unterschreitet, und die Pumprate des pulsatilen Fluidflusses und/oder den Saugdruck anschließend zu verringern.

Somit werden Ereignisse wie das Ansaugen der Herzwand oder das Kollabieren von Hohlvenen vermeidbar, ohne dass die Pumprate des pulsatilen Fluidflusses bzw. der Saugdruck übermäßig verringert wird.

Das Pumpensystem kann weiterhin eine zweite Membranfluidpumpe umfassen, die ebenfalls mit dem Herzen und/oder mindestens einem mit dem Herzen verbundenen Blutgefäß fluidisch verbindbar und zum Erzeugen eines pulsatilen Fluidflusses zur Unterstützung einer Herzaktivität des Herzens eingerichtet und ferner mittels einer Druckleitung mit der Arbeitsdruckquelle oder einer zweiten Arbeitsdruckquelle verbunden ist, wobei die Steuereinheit zum Regeln der zweiten Membranfluidpumpe in gleicher Weise wie zum Regeln der ersten Membranfluidpumpe eingerichtet sein kann.

Das zwei Membranfluidpumpen umfassende Pumpensystem eignet sich besonders als biventrikuläres VAD (BVAD), womit die Vorteile des Pumpensystems auch für Patienten erschlossen werden, die ein BVAD benötigen.

Das Pumpensystem kann als stationäres oder mobiles System ausgebildet sein.

Die vorgeschlagene Steuereinheit ist für die Verwendung mit dem vorgeschlagenen Pumpensystem vorgesehen. Die Steuereinheit ist also zum Regeln des Arbeitsdrucks und ferner dazu eingerichtet, den Zeitversatz zu bestimmen,

basierend auf dem Zeitversatz und/oder dem ersten Kanülenflusssignal und/oder dem Arbeitsdrucksignal den hämodynamischen Parametersatz zu bestimmen und

basierend auf der Regelvorgabe den Zeitversatz derart zu bestimmen, dass die Regelvorgabe erreicht wird.

Die Steuereinheit kann zur Verwendung mit verschiedenen Membranfluidpumpen und/oder Arbeitsdruckquellen, beispielsweise Membranfluidpumpen verschiedener Größe (d. h. insbesondere mit verschiedenen Füllvolumen) und/oder Arbeitsdruckquellen, wahlweise umfassend eine Kolbenpumpe oder einen Pneumatikschaltkreis, eingerichtet sein.

Die Steuereinheit kann als selbstständige Einheit ausgebildet sein, oder sie kann beispielsweise als nachrüstbare Softwarekomponente einer bestehenden Steuereinheit, einer mobilen Datenverarbeitungsgeräts und/oder eines Workstation-Rechners vorgesehen sein.

Die Steuereinheit kann gemäß der oben im Zusammenhang mit dem Pumpensystem erwähnten Merkmale fortgebildet werden.

Das vorgeschlagene Verfahren zum Betreiben eines Pumpensystems der oben beschriebenen Art umfasst die Schritte:

Erfassen des ersten Kanülenflusssignals und des Arbeitsdrucksignals, Bestimmen des Zeitversatzes und/oder des Entleerungszeitversatzes für den ersten Pumpzyklus und den ersten Herzzyklus,

Bestimmen des hämodynamischen Parametersatzes basierend auf dem Zeitversatz und/oder dem ersten Kanülenflusssignal und/oder dem Arbeitsdrucksignal,

Bestimmen der Zeitvorgabe basierend auf der Regelvorgabe derart, dass die Regelvorgabe erreicht wird.

Das Verfahren stellt also eine vorteilhafte Verwendung des beschriebenen Pumpensystems dar. Das Verfahren kann, je nach Beschaffenheit des Pumpensystems und den Erfordernissen der Pumpenverwendung, weitere Schritte umfassen, die sich beispielsweise auf den oben im Zusammenhang mit dem Pumpensystem erwähnten Merkmalen ergeben können.

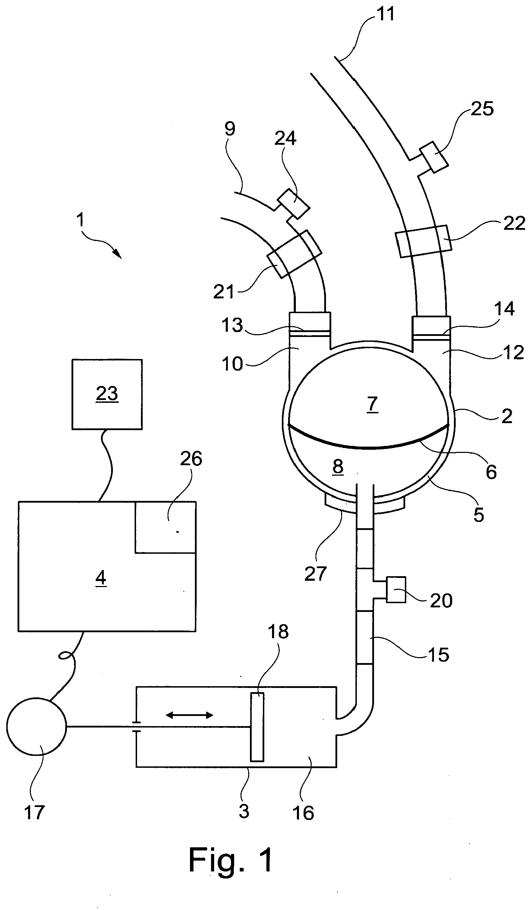

Ausführungsbeispiele des beschriebenen Pumpensystems und des beschriebenen Verfahrens zum Betreiben eines Pumpensystems werden nachfolgend anhand Fig. 1 bis Fig. 10 erläutert. Dabei werden verschiedene wesentliche oder auch vorteilhafte weiterbildende Elemente im Rahmen jeweils eines konkreten Beispiels genannt, wobei auch einzelne dieser Elemente als solche zur Weiterbildung des Pumpensystems, der Steuereinheit und/oder des Verfahrens - auch herausgelöst aus dem Kontext des jeweiligen Beispiels und weiterer Merkmale des jeweiligen Beispiels - verwendet werden können. Dabei zeigen

Fig. 1 eine schematische Darstellung eines Pumpensystems,

Fig. 2 eine graphische Darstellung von Ergebnissen einer ersten Simulation,

Fig. 3 eine graphische Darstellung weiterer Ergebnisse der ersten Simulation,

Fig. 4 eine graphische Darstellung weiterer Ergebnisse der ersten Simulation,

Fig. 5 eine graphische Darstellung weiterer Ergebnisse der ersten Simulation,

Fig. 6a eine graphische Darstellung von Ergebnissen einer zweiten Simulation,

Fig. 6b eine graphische Darstellung von Ergebnissen einer dritten Simulation,

Fig. 6c eine graphische Darstellung von Ergebnissen einer vierten Simulation,

Fig. 7 eine schematische Darstellung eines Beispiels eines Bestimmens hämodynamischer Parameter,

Fig. 8 eine schematische Darstellung eines ersten Beispiels einer Synchronisation,

Fig. 9 eine schematische Darstellung eines zweiten Beispiels einer Synchronisation,

Fig. 10 eine schematische Darstellung eines dritten Beispiels einer Synchronisation.

Wiederkehrende und ähnliche Merkmale verschiedener Ausführungsbeispiele werden in den Figuren und der nachfolgenden Beschreibung jeweils mit denselben Bezugzeichen gekennzeichnet, wobei die Bezugzeichen teilweise ausgelassen werden, wenn die bezeichneten Merkmale in Bezug auf eine bestimmte Zeichnung nicht erwähnt werden.

Das in Fig. 1 gezeigte Pumpensystem 1 umfasst eine Membranfluidpumpe 2, eine Arbeitsdruckquelle 3 und eine Steuereinheit 4.

Die Membranfluidpumpe 2 umfasst ein Gehäuse 5, in dessen Inneren ein Hohlraum ausgebildet ist, der durch eine auslenkbare Membran 6 in eine Pumpfluidkammer 7 und eine Arbeitsfluidkammer 8 geteilt ist.

Die Pumpfluidkammer 7 weist einen mit einer Einlasskanüle 9 verbundenen Fluideinlass 10 sowie einen mit einer Auslasskanüle 11 verbundenen Fluidauslass 12 auf.

Die Arbeitsfluidkammer 8 ist mit Luft als Arbeitsfluid füllbar und mittels einer Druckleitung 15 mit der Arbeitsdruckquelle 3 so verbunden, dass mittels der Arbeitsdruckquelle 3 ein Überdruck und/oder ein Unterdruck in der Arbeitsfluidkammer 8 zum Auslenken der Membran 6 erzeugt werden kann, wodurch die Arbeitsdruckquelle 3 also zum Bereitstellen eines Arbeitsdrucks zum Antreiben der Membranfluidpumpe 2 eingerichtet ist.

Die Arbeitsdruckquelle 3 ist im Beispiel eine Kolbenpumpe, umfassend einen in einem Arbeitsraum 16 bewegbaren und von einem Elektromotor 17 angetriebenen Arbeitskolben 18. Die Arbeitsdruckquelle 3 kann alternativ eine andere Art von Pumpe oder ein Pneumatikschaltkreis, beispielweise ein Pneumatikschaltkreis umfassend einen Kompressor, einen Überdruckbehälter und einen Unterdruckbehälter, sein. Das Arbeitsfluid kann auch ein anderes Gas oder auch eine Flüssigkeit sein.

Die Steuereinheit 4 ist eingerichtet zum Regeln des Arbeitsdrucks. Zu diesem Zweck ist sie mit dem Motor 17 der Arbeitsdruckquelle 3 über eine Steuerleitung 19 verbunden. Das Pumpensystem 1 umfasst weitere Leitungen, etwa Steuer- und Kommunikationsleitungen, beispielsweise zwischen der Steuereinheit 4 und den verschiedenen Sensoren, die zur Vereinfachung nicht abgebildet sind.

An der Druckleitung 15 ist ein Arbeitsdrucksensor 20 angeordnet, eingerichtet zum Erfassen eines Arbeitsdrucksignals, das dem Arbeitsdruck in der Druckleitung 15 entspricht.

Das mittels des Arbeitsdrucksensors 20 erfasste Arbeitsdrucksignal wird von der Steuereinheit 4 als Überwachungs- und Rückkopplungssignal zum Regeln des Arbeitsdrucks und somit des pulsatilen Fluidflusses verwendet.

Die Membranfluidpumpe 2 umfasst ein im Fluideinlass 10 angeordnetes Einlassventil 13 und ein im Fluidauslass 12 angeordnetes Auslassventil 14. Die Ventile 13 und 14 sind Einwegventile, so dass ohne eine aktive Steuerung erreicht wird, dass ein Fluid nur durch den Einlass 10 einströmen und nur durch den Auslass 12 ausströmen kann. Alternativ können das Einlassventil 13 und/oder das Auslassventil 14 aktuierte Ventile sein, die mittels der Steuereinrichtung 4 so steuerbar sind, dass das Fluid im Betrieb durch den Einlass 10 einströmen und durch den Auslass 12 ausströmen kann.

Die Membranfluidpumpe 2 ist mittels der Einlasskanüle 9 und der Auslasskanüle 11 mit einem Herzen und/oder mindestens einem mit dem Herzen verbundenen Blutgefäß fluidisch verbindbar. Beispielsweise kann die Einlasskanüle 9 mit einem linken Ventrikel des Herzens, die Auslasskanüle 11 mit einer Aorta verbindbar sein, wodurch das Pumpensystem als linksseitiges VAD (LVAD) einsetzbar ist. Alternativ kann die Einlasskanüle 9 beispielsweise mit einem rechten Ventrikel des Herzens, die Auslasskanüle 11 mit einer Pulmonalarterie verbindbar sein, wodurch das Pumpensystem als rechtsseitiges VAD (RVAD) einsetzbar ist. Beispielsweise für die Behandlung von Failing-Fontan-Patienten kann die Einlasskanüle mit mindestens einer Hohlvene, die Auslasskanüle mit einer Pulmonalarterie verbindbar sein.

Das Pumpensystem 1 weist einen ersten Flusssensor 21 und einen zweiten Flusssensor 22 auf. Der erste Flusssensor 21 ist an der Einlasskanüle 9 angeordnet und zum Erfassen eines ersten Kanülenflusssignals, das einem Einlassfluss in der Einlasskanüle 9 entspricht, eingerichtet. Der zweite Flusssensor 22 ist an der Auslasskanüle 11 angeordnet und zum Erfassen eines zweiten Kanülenflusssignals, das einem Auslassfluss in der Auslasskanüle 11 entspricht, eingerichtet. Der erste Flusssensor 21 und der zweite Flusssensor 22 sind Ultraschallflusssensoren, können aber auch andere Arten von Flusssensoren sein, beispielsweise Hall-Effekt-Sensoren. Das Pumpensystem 1 kann auch nur einen einzigen Flusssensor aufweisen, der beispielsweise an der Einlasskanüle 9 oder an der Auslasskanüle 11 angeordnet sein kann, oder es kann mehr als zwei Flusssensoren an verschiedenen Stellen des durchflossenen Bereichs aufweisen.

Die Membranfluidpumpe 2 ist zum Erzeugen eines pulsatilen Fluidflusses zur Unterstützung einer Herzaktivität des Herzens eingerichtet. Der pulsatile Fluidfluss wird durch Auslenken der Membran 6 in einander entgegengesetzte Richtungen erzeugt.

Hierbei wird in jedem Pumpzyklus zunächst mittels der Arbeitsdruckquelle 3 ein Unterdruck in der Arbeitsfluidkammer 8 erzeugt, indem der Arbeitsraum 16 durch Bewegen des Arbeitskolbens 18 mittels des Elektromotors 17 vergrößert wird. Hierbei schließt sich das Auslassventil 14, das Einlassventil 13 öffnet sich. Durch den Unterdruck und das geöffnete Einlassventil 13 strömt Blut durch den Fluideinlass 10 in die Pumpfluidkammer 7 ein (Füllungsphase). Ist diese gefüllt, d. h. ist die Membran 6 in einer ersten Endlage, so wird der Arbeitsdruckquelle 3 (durch Bewegen des Arbeitskolbens 18 in eine entgegengesetzte Richtung) ein Überdruck erzeugt, das Einlassventil 13 schließt sich und das Auslassventil 14 öffnet sich, so dass Blut durch den Auslass 12 aus der Pumpfluidkammer 7 verdrängt wird (Entleerungsphase), bis die Pumpfluidkammer 7 entleert ist. (Die Endlage der Membran 6 kann etwa mittels eines Integrals des ersten Kanülenflusssignals und/oder anhand eines Abflachens des ersten Kanülenflusssignals erfasst und/oder durch verschiedene Steuerverfahren sichergestellt werden kann - vgl. hierzu die Druckschrift EP 3 536 955 A1.)

Anschließend kann der nächste Pumpzyklus beginnen. Beginn und Dauer der Füllungsphase und der Entleerungsphase werden jeweils von der Steuereinheit 4 als Zeitvorgabe bestimmt (Beispiele hierfür werden weiter unten beschrieben). Entsprechend dieser Zeitvorgabe kann zwischen den aufeinanderfolgenden Füllungs- und Entleerungsphasen eine Phase mit einem Arbeitsdruck der (zur Erhöhung der Messempfindlichkeit) zwischen dem Füll- und dem Entleerungsdruck liegt, etwa die oben erwähnte Messphase, vorgesehen sein.

Das Pumpensystem 1 umfasst weiterhin EKG-Sensoren 23, eingerichtet zum Erfassen eines EKG-Signals, das einer elektrischen Aktivität des Herzens entspricht, einen ersten Kanülendrucksensor 24, eingerichtet zum Erfassen eines ersten Kanülendrucksignals, das einem Einlassdruck in der Einlasskanüle 9 entspricht, sowie einen zweiten Kanülendrucksensor 25, eingerichtet zum Erfassen eines zweiten Kanülendrucksignals, das einem Auslassdruck in der Auslasskanüle 11 entspricht. Das Pumpensystem 1 kann beispielsweise auch nur einen einzigen Kanülendrucksensor aufweisen, der beispielsweise an der Einlasskanüle 9 oder an der Auslasskanüle 11 angeordnet sein kann.

Die Steuereinheit 4 weist ferner eine Anzeigeeinheit 26 auf, die zum Anzeigen verschiedener Systemparameter, Einstellvorschläge, hämodynamischer Parameter und Steueroptionen eingerichtet ist.

Die Membranfluidpumpe 2 umfasst weiterhin einen Beschleunigungssensor 27, eingerichtet zum Erfassen eines einer Patientenaktivität entsprechenden Beschleunigungssignals.

Die Steuereinheit 4 ist dazu eingerichtet, einen Zeitversatz zwischen einem ersten Zeitpunkt, der während eines ersten Pumpzyklus auftritt, und einem zweiten Zeitpunkt, der während eines ersten Herzzyklus auftritt, zu bestimmen. Das Bestimmen des Zeitversatzes wird nachfolgend anhand verschiedener Beispiele beschrieben. Hierbei werden in den verschiedenen Beispielen verschiedene Sensorsignale zum Bestimmen des Zeitversatzes verwendet. Die Steuereinheit 4 kann zum Bestimmen des Zeitversatzes beispielsweise entsprechend einem oder mehrerer der Beispiele eingerichtet sein (auch abhängig davon, welche Sensoren das Pumpensystem 1 beispielsweise in unterschiedlichen Ausführungsformen aufweist) und kann alternativ oder zusätzlich auch dazu eingerichtet sein, den Zeitversatz auf andere Weise oder basierend auf anderen Sensorsignalen zu bestimmen.

In einem ersten Beispiel wird der Zeitversatz basierend auf dem ersten Kanülenflusssignal, das dem Einlassflusszeitverlauf $Q(t)$ entspricht, bestimmt. Um dieses Beispiel zu verdeutlichen, wurden ein beispielhafter Einlassflussverlauf $Q(t)$ im Einlass 10 der Membranfluidpumpe 2 und ein beispielhafter Ventrikeldruckverlauf $p_{LV}(t)$ eines mit der Membranfluidpumpe 2 verbundenen Ventrikels durch eine erste Simulation des Pumpensystems 1 berechnet. Die erste Simulation beruht auf den folgenden Parametern und Annahmen: Wenn dem Ventrikel das komplette Füllvolumen der angeschlossenen Membranfluidpumpe 2 entzogen wurde, baut er nur die Hälfte des Druckes auf, den er ohne Entzug des Füllvolumens aufbauen würde. Der Zeitverlauf des Druckaufbaus und-abfalls werden durch die skalierte Kosinusfunktion approximiert. Die Systole dauert 50 % des Herzschlags. Der Druckanstieg dauert 70 % der Systole. Der Arbeitsdruck hat einen trapezförmigen Zeitverlauf. Das Füllvolumen beträgt 80 ml, der Ventrikeldruck variiert mit einer maximalen Druckänderungsrate von 10 bis 120 mmHg/s, der Aortendruck beträgt 100 mmHg, die Herzrate beträgt 78 bpm, die Pumprate beträgt 82 bpm.

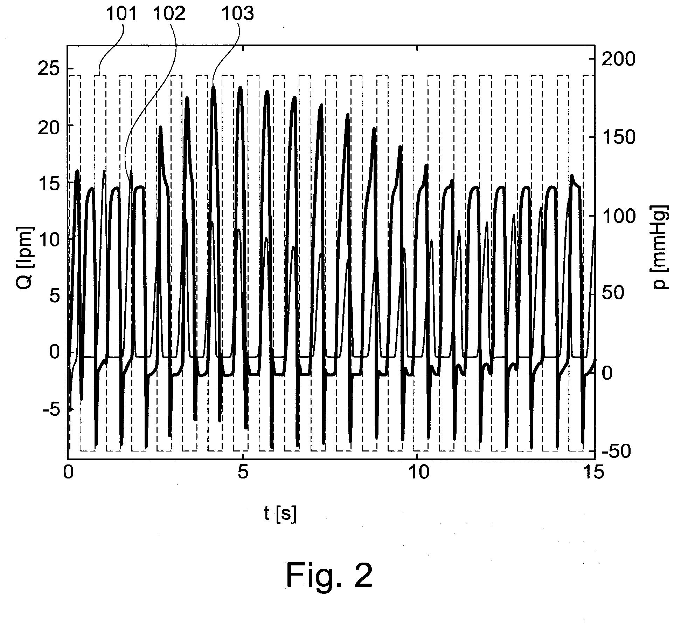

Der Graph in Fig. 2 zeigt beispielhafte Zeitverläufe der ersten Simulation. Kurve 101 zeigt den Arbeitsdruckzeitverlauf $p_A(t)$, Kurve 102 den Ventrikeldruckzeitverlauf $p_{LV}(t)$, Kurve 103 den Einlassflusszeitverlauf $Q(t)$. Hierbei ist zu erkennen, dass der Einlassfluss erhöht ist, wenn die Systole während der Füllungsphase der Membranfluidpumpe 2 stattfindet (der Zeitversatz zwischen den Kurven 102 und 103 verschiebt sich aufgrund des Unterschieds zwischen Herzrate und Pumprate in Abhängigkeit von der Zeit t. Der Druckaufbau durch den Ventrikel nimmt ab, wenn das Ventrikelvolumen in der Systole reduziert ist.

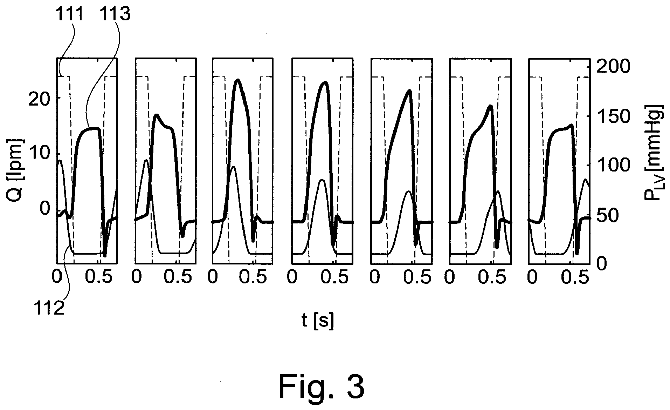

Um das Bestimmen des Zeitversatzes zu verdeutlichen, wurden simulierte Daten wie die, die in Fig. 2 gezeigt sind, in sieben Gruppen aufgeteilt. Jede Gruppe entspricht einem gleichgroßen Bereich des Zeitversatzes. In der ersten Gruppe liegt der Ventrikelspitzendruck maximal weit vor der Mitte der Füllphase. In der mittleren Gruppe stimmen die beiden Zeitpunkte überein. Die sieben Einzelgraphen in Fig. 3 zeigen die in den Gruppen gebildeten Mittelwerte über den Arbeitsdruckzeitverlauf (Kurven 111), den Ventrikeldruckzeitverlauf (Kurven 112) und den Einlassflusszeitverlauf (Kurven 113) aus der ersten Simulation.

An den Kurven 113 ist zu erkennen, wie sich die Form des Einlassflussverlaufs bei geändertem Zeitversatz ändert. Für eine quantitative Bestimmung des Zeitversatzes wird zunächst die Schiefe des Einlassflussverlaufs gemäß der Gleichung

$$s = \int_{t_S}^{t_E} \left( \frac{Q(t) - \langle Q \rangle}{\sigma_Q} \right)^3 dt$$

berechnet. Dabei ist s die Schiefe, $Q(t)$ der Einlassflusszeitverlauf, t ein Zeitparameter, $t_S$ der Zeitpunkt, zu dem $Q(t)$

zum letzten Mal vor dem Erreichen eines Maximalflusses $Q_{max}$ weniger als 20 % von $Q_{max}$ beträgt, $t_E$ der Zeitpunkt, zu dem Q(t) zum letzten Mal seit Erreichen von $Q_{max}$ weniger als 20 % von $Q_{max}$ beträgt, <Q> der Mittelwert von Q(t) im Zeitintervall von $t_S$ bis $t_E$, $\sigma_Q$ die Standardabweichung von Q(t) im Zeitintervall von $t_S$ bis $t_E$. Ferner wird der quadratische Mittelwert r des Einlassflussverlaufs im Zeitintervall von $t_S$ bis $t_E$ als

$$r = \sqrt{\frac{1}{t_1 - t_0} \int_{t_0}^{t_1} Q(t)^2 \, dt}$$

berechnet.

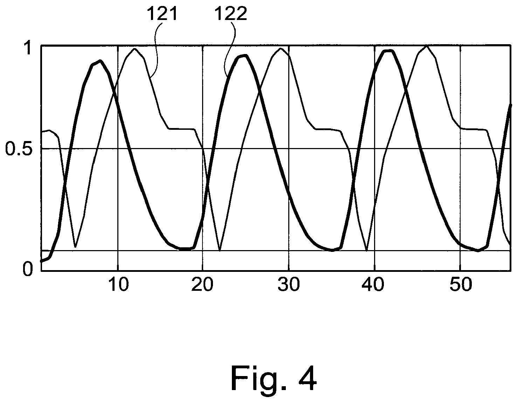

Fig. 4 zeigt einen Zeitverlauf der Schiefe s (Kurve 121) und des quadratischen Mittelwerts r (Kurve 122) über mehrere Pumpzyklen (x-Achse) der ersten Simulation, wobei r und s zur besseren Darstellung auf das Intervall von 0 bis 1 normiert sind.

Zur weiteren Berechnung wird eine komplexe Zahl c definiert als c = r + i s, wobei i die imaginäre Einheit ist. Eine relative Phasenlage p zwischen dem Pumpzyklus und dem Herzzyklus kann dann geschätzt werden als p = arg(c) oder p = arg(c·$e^{i\alpha}$), wobei die optionale Multiplikation mit $e^{i\alpha}$ die Phase lediglich um einen gewünschten Winkeloffset $\alpha$ verschiebt.

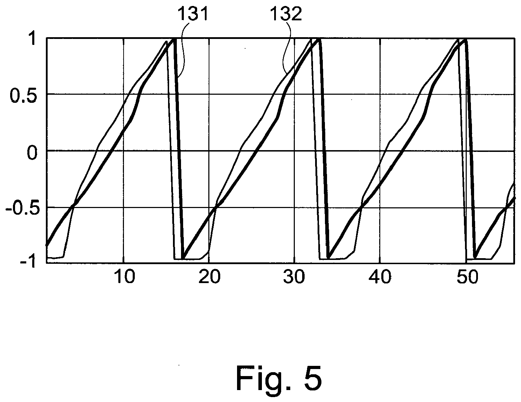

Fig. 5 zeigt einen Zeitverlauf der tatsächlichen relativen Phasenlage gemäß der Simulation (Kurve 131) sowie der als p = arg(c · $e^{i\alpha}$) bestimmten relative Phasenlage (Kurve 132), jeweils durch Division durch die Kreiszahl $\pi$ auf das Intervall von -1 bis 1 normiert, über mehrere Pumpzyklen (x-Achse) der ersten Simulation. Aus der so normierten relativen Phasenlage p/$\pi$ kann der Zeitversatz T als T = p · $\tau$/2$\pi$ geschätzt werden, wobei $\tau$ die Periodendauer des pulsatilen Fluidflusses ist. Zusammenfassend ergibt sich T also als

$$T = \frac{\tau}{2\pi} \arg\left(\left[\sqrt{\frac{1}{t_1 - t_0} \int_{t_0}^{t_1} Q(t)^2 \, dt} + i \int_{t_S}^{t_E} \left(\frac{Q(t) - \langle Q \rangle}{\sigma_Q}\right)^3 \, dt\right] e^{i\alpha}\right).$$

In Fig. 5 ist erkennbar, dass die Bestimmung des Zeitversatzes über einen weiten Bereich gute Ergebnisse liefert. Lediglich im Bereich des in Fig. 4 erkennbaren Plateaus der Kenngrößen r und s wird die Bestimmung ungenau, da der Einlassfluss hier gegenüber Änderungen der Phasenlage unempfindlich ist. Die Steuereinheit 4 kann dazu eingerichtet sein, die Zeitvorgabe so einzustellen, dass wenigstens temporär eine für die Bestimmung des Zeitversatzes günstige relative Phasenlage erzielt wird, insbesondere so, dass der Beginn oder das Ende der Entleerungsphase des ersten Herzzyklus während der Füllphase des ersten Pumpzyklus auftritt. Damit kann die genannte Unempfindlichkeit umgangen werden.

In einem zweiten Beispiel wird der Zeitversatz ebenfalls basierend auf dem ersten Kanülenflusssignal, das dem Einlassflusszeitverlauf Q(t) entspricht, bestimmt. Im ersten Beispiel konnte der Zeitversatz aus zwei Kenngrößen des Einlassflusszeitverlaufs, s und r, bestimmt werden. Wie aus Fig. 4 ersichtlich, würde das Betrachten einer einzigen der beiden Größen noch keine eindeutige Bestimmung der Phasenlage erlauben, da jeder Wert von s und r bei zwei verschiedenen Phasenlagen auftritt. Jedoch kann die Steuereinheit 4 dazu eingerichtet sein, die Pumprate bzw. Periodendauer über mehrere Pumpzyklen zu variieren, so dass das Vorzeichen der Ableitung von r und/oder s bestimmbar ist, womit ebenfalls eine eindeutige Bestimmung der Phasenlage und damit des Zeitversatzes ermöglicht wird.

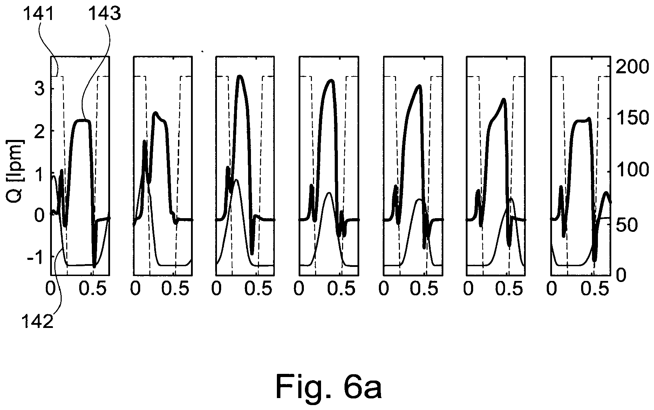

Fig. 6a bis 6c zeigen Ergebnisse weiterer Simulationen, deren Vorgehen, Annahmen und Parameter mit der ersten Simulation bis auf die im Folgenden genannten Abweichungen übereinstimmen, mit denen überprüft werden soll, ob Laständerungen und andere Parameteränderungen die Bestimmung der Phasenlage stören .

Fig. 6a zeigt die (über analog zu Fig. 3 gebildete sieben Gruppen) gemittelten Zeitverläufe von Arbeitsdruck (Kurven 141), Ventrikeldruck (Kurven 142) und Einlassfluss (Kurven 143) einer zweiten Simulation. Das Füllvolumen beträgt hierbei 10 ml, die Herzrate beträgt 75 bpm.

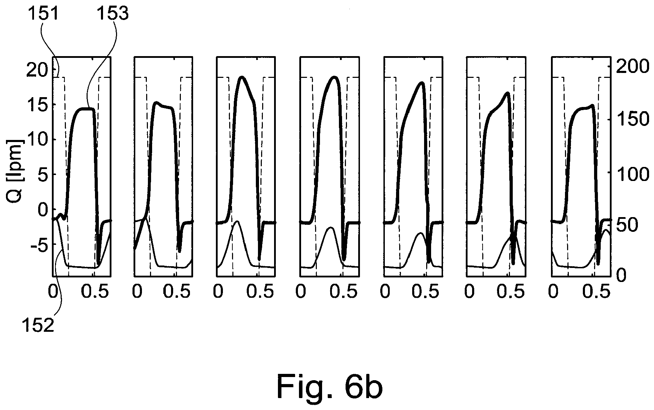

Fig. 6b zeigt die (über analog zu Fig. 3 gebildete sieben Gruppen) gemittelten Zeitverläufe von Arbeitsdruck (Kurven 151), Ventrikeldruck (Kurven 152) und Einlassfluss (Kurven 153) einer dritten Simulation. Der Ventrikeldruck variiert hierbei mit einer maximalen Druckänderungsrate von 8 bis 60 mmHg/s.

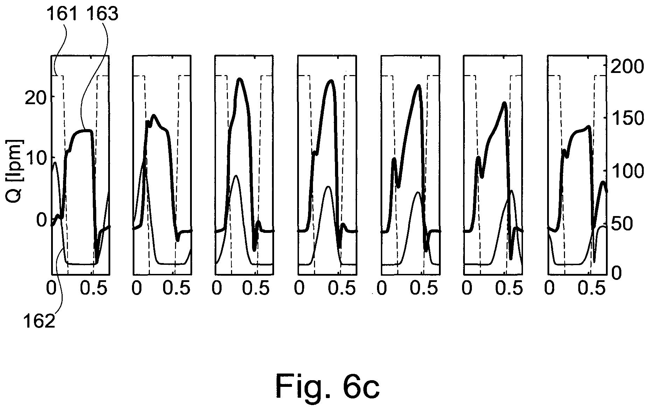

Fig. 6c zeigt die (über analog zu Fig. 3 gebildete sieben Gruppen) gemittelten Zeitverläufe von Arbeitsdruck (Kurven 161), Ventrikeldruck (Kurven 162) und Einlassfluss (Kurven 163) einer vierten Simulation. Der Aortendruck beträgt hierbei 70 mmHg.

In Fig. 6a-6c ist gut zu erkennen, dass die Abhängigkeit der Einlassflusskurven von der Phasenlage qualitativ ähnlich ist wie in Fig. 3. Der oben angegebene Formalismus ist auch hier anwendbar. Das Verfahren ist also robust gegenüber Laständerungen. Es kann jedoch vorteilhaft sein, die Größen s und r für jeden Parametersatz neu zu normalisieren bzw. zu kalibrieren, um Abweichungen zu minimieren. Im Betrieb ist es zusätzlich möglich, die Kalibrierung regelmäßig zu wiederholen, um auch nach großen Laständerungen weiterhin zuverlässig zu synchronisieren. Die Abbildung auf die Phasenlage kann auch auf andere Weise als oben beschrieben bestimmt werden, beispielsweise mittels eines neuronalen Netzes, für das die Flussverläufe gemäß Fig. 3 und Fig. 6a-6c als Eingänge, die Phasenlage als Ausgang dienen könnte.

In einem dritten Beispiel wird der Zeitversatz basierend auf dem EKG-Signal bestimmt. Die Steuereinheit 4 ist dazu eingerichtet, einen QRS-Komplex als Anteil des EKG-Signals zu erkennen. Dieser entspricht dem Beginn der Entleerungsphase des ersten Herzzyklus. Da der Beginn der Entleerungsphase des ersten Pumpzyklus bekannt ist, kann damit unmittelbar der Zeitversatz als Entleerungszeitversatz bestimmt werden. Die Steuereinheit 4 kann zusätzlich oder alternativ zum Erkennen einer T-Welle und/oder einer P-Welle als Anteil des EKG-Signals eingerichtet sein.

Die Steuereinheit 4 ist ferner dazu eingerichtet, basierend auf dem Zeitversatz und/oder dem ersten Kanülenflusssignal und/oder dem Arbeitsdrucksignal einen hämodynamischen Parametersatz, umfassend mehrere hämodynamische Parameter, zu bestimmen. Das Bestimmen hämodynamischer Parameter wird nachfolgend anhand verschiedener Beispiele beschrieben. Hierbei werden in den verschiedenen Beispielen verschiedene Sensorsignale zum Bestimmen verschiedener hämodynamischer Parameter des hämodynamischen Parametersatzes verwendet. Die Steuereinheit 4 kann zum Bestimmen hämodynamischer Parameter beispielsweise entsprechend einem oder mehrerer der Beispiele eingerichtet sein (auch abhängig davon, welche Sensoren das Pumpensystem 1 beispielsweise in unterschiedlichen Ausführungsformen aufweist) und kann alternativ oder zusätzlich auch dazu eingerichtet sein, dieselben oder weitere hämodynamische Parameter auf andere Weise oder basierend auf anderen Sensorsignalen zu bestimmen.

In einem ersten Beispiel werden verschiedene hämodynamische Parameter basierend auf dem ersten Kanülenflusssignal und/oder dem zweiten Kanülenflusssignal bestimmt. Hierbei ist die Periodendauer des pulsatilen Fluidflusses zunächst so eingestellt, dass eine kontinuierlich veränderliche relative Phasenlage auftritt (ähnlich wie etwa in der ersten Simulation, vgl. Fig. 2). Die Kontraktilität kann dann als Maximalwert des ersten Kanülenflusssignals pro Pumpzyklus bestimmt werden, die Vorlast als Minimalwert des ersten Kanülenflusssignals pro Pumpzyklus, die Nachlast als Maximalwert des zweiten Kanülenflusssignals pro Pumpzyklus. Die genannten Maximal- und Minimalwerte können schwache, stark gestörte bzw. verrauschte Signale sein. Die Steuereinheit 4 kann dazu eingerichtet sein, zum besseren Bestimmen schwacher, stark gestörter bzw. verrauschter Signale zeitliche Mittelwerte über einen längeren Zeitraum, beispielsweise über Nacht, zu bestimmen, d.h. eine Langzeitmittelung vorzunehmen. Die Langzeitmittelung über Nacht ist vorteilhaft, weil so zusätzlich die Lage der Membranfluidpumpe in Bezug auf das Herz/den Kreislauf des Patienten relativ konstant gehalten wird. Alternativ oder zusätzlich zur Messung über Nacht kann die Steuereinheit 4 zum Bestimmen mindestens eines Lageparameters eines Patienten (etwa wie weiter oben beschrieben) und zum Korrigieren der Langzeitmittelung anhand des Lageparameters eingerichtet sein.

In einem zweiten Beispiel werden, wie im ersten Beispiel, verschiedene hämodynamische Parameter basierend auf dem ersten Kanülenflusssignal und/oder dem zweiten Kanülenflusssignal, zusätzlich aber auch basierend auf dem EKG-Signal bestimmt. Das EKG-Signal wird hierbei verwendet, um das erste und/oder zweite Kanülenflussignal genau während der Diastole und/oder der Systole des Herzzyklus zu erfassen. Auch in diesem Beispiel kann und sollte die Steuereinheit 4 zum Vornehmen einer Langzeitmittelung und/oder zum Korrigieren der Langzeitmittelung anhand des Lageparameters eingerichtet sein.

In einem dritten Beispiel werden verschiedene hämodynamische Parameter basierend auf dem ersten und zweiten Kanülenflusssignal sowie dem EKG-Signal bestimmt. Hierbei wird ausgenutzt, dass anhand des EKG-Signals eine genaue Verzögerung der Füllphase bzw. der Entleerungsphase des pulsatilen Fluidflusses eingestellt werden kann. In einem ersten Schritt wird hierbei der Beginn der Füllphase des pulsatilen Fluidflusses über mehrere Pumpzyklen gegenüber dem Beginn der Füllphase der Herzaktivität schrittweise verzögert. Mit der so gewonnen Information kann zunächst eine Kalibrierung vorgenommen werden, bei welcher der Arbeitsdruck an die Lage der Membranfluidpumpe 2 angepasst wird. Dazu wird beispielsweise der Entleerungsdruck so eingestellt, dass der resultierende Verlauf des zweiten Kanülenflusssignals bei einem minimalen Aortendruck einem zuvor gespeicherten Verlauf des zweiten Kanülenflusssignals entspricht. Nach der Kalibrierung ist es möglich, eine Änderungsrate des ersten Kanülenflusssignals in der Systole als Maß für die Kontraktilität zu bestimmen. Aus der Variation des zweiten Kanülenflusssignals kann eine Kurvenform des Aortendrucks bestimmt werden, woraus wiederum eine Öffnungsdauer der Aortenklappe (als Dauer für den Druckaufbau), ein Kreislaufwiderstand (als Dauer für den Druckabbau) und die Kontraktilität (als Steigung des Druckaufbaus) bestimmbar sind.

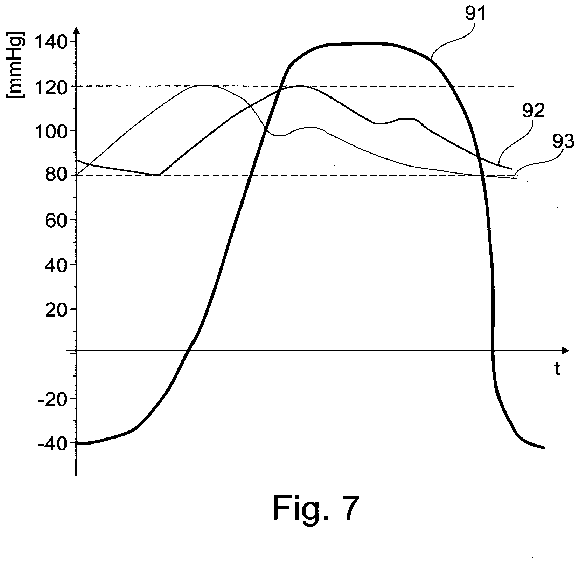

Fig. 7 zeigt für diese Art der Parameterbestimmung einen beispielhaften Zeitverlauf des Pumpendrucks (Kurve 91), des Aortendrucks für eine erste Verzögerung (Kurve 92) und des Aortendrucks für eine zweite Verzögerung (Kurve 93). Das gemessene zweite Kanülenflusssignal beginnt, wenn der Pumpendruck größer ist als der Aortendruck, weshalb über die Aufnahme einer Vielzahl von Messwerten des zweiten Kanülenflusssignals der Aortendruck näherungsweise bestimmt werden kann.

In einem vierten Beispiel werden verschiedene hämodynamische Parameter basierend auf dem ersten Kanülendrucksignal bestimmt. Die Vorlast wird als enddiastolischer Druck bestimmt. Die Kontraktilität wird aus einer volumenabhängigen Änderungsrate des systolischen Drucks bestimmt. Die Nachlast wird als über einen Herzzyklus bestimmter Maximaldruck bestimmt.

Die Steuereinheit 4 ist weiterhin dazu eingerichtet, basierend auf einer Regelvorgabe als Vorgabe für den hämodynamischen Parametersatz und/oder für den Zeitversatz eine Zeitvorgabe als Vorgabe für einen Beginn und/oder eine Dauer der Füllungsphase und/oder der Entleerungsphase des ersten Pumpzyklus und/oder eines zweiten Pumpzyklus, der zeitlich nach dem ersten Pumpzyklus auftritt, derart zu bestimmen, dass die Regelvorgabe erreicht wird.

Die Steuereinheit 4 ist dazu eingerichtet, den Arbeitsdruck basierend auf der Zeitvorgabe so zu regeln, dass die Regelvorgabe erreicht wird. Alternativ oder zusätzlich kann die Anzeigeeinheit 26 zum Anzeigen einer basierend auf der Zeitvorgabe bestimmten vorgeschlagenen Einstellungsänderung eingerichtet sein, die dann von dem Patienten oder medizinischem Personal ausgewählt werden kann.

Die Regelvorgabe kann verschiedene Regelziele bezwecken. Ein mögliches Regelziel besteht darin, den Grad der Herzunterstützung, insbesondere den über mehrere Pumpzyklen gemittelten mittleren Blutfluss durch die Membranfluidpumpe, an physiologische Änderungen oder Ereignisse anzupassen, wie oben beschrieben.

Nachfolgend wird das weitere Regelziel der Synchronisation, also das Einstellen einer vorgegebenen relativen Phasenlage des pulsatilen Fluidflusses in Bezug auf die Herzaktivität, anhand verschiedener Beispiele beschrieben.

Bei der Synchronisation ist in jedem Fall vorgesehen, dass die Zeitvorgabe eine Vorgabe für den Beginn und/oder die Dauer der Füllungsphase und/oder der Entleerungsphase des ersten und/oder zweiten Pumpzyklus ist und dass die Regelvorgabe eine Vorgabe für den Zeitversatz derart ist, dass die vorgegebene relative Phasenlage des pulsatilen Fluidflusses in Bezug auf die Herzaktivität erreicht wird.

In einem ersten Beispiel ist die Steuereinheit 4 zum Erzielen der Synchronisation basierend auf dem ersten Kanülenflusssignal mittels eines schnellen Verfahrens eingerichtet, bei dem die Synchronisation noch innerhalb des ersten Herzzyklus basierend auf dem bestimmten Zeitversatz erzielt wird.

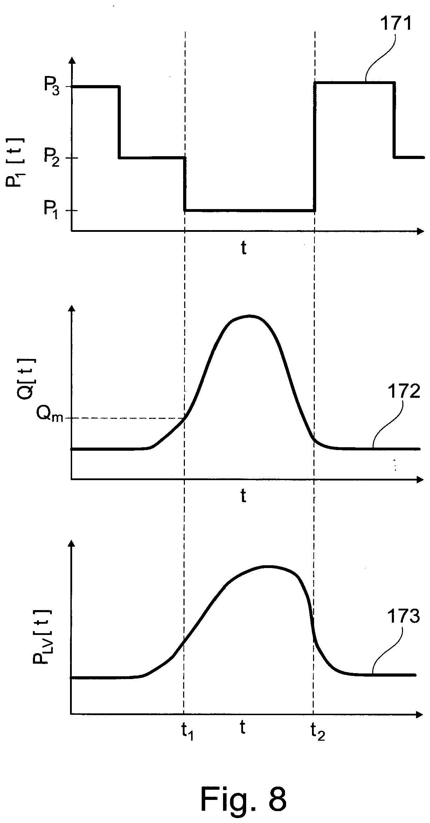

Diese Art der Synchronisation ist in Fig. 8 illustriert, wobei hier ein Gegenpuls als relative Phasenlage eingestellt wird. Fig. 8 zeigt Zeitverläufe des Arbeitsdrucks $p_A(t)$ (Kurve 171), des ersten Kanülenflusssignals Q(t) (Kurve 172) und des Ventrikeldrucks $p_{LV}(t)$ im linken Ventrikel (Kurve 173).

Hierbei wird zunächst ein Arbeitsdruck $p_2$ eingestellt, der etwas über dem Einlassdruck in der Diastole liegt. Der Beginn der Systole wird im ersten Kanülenflusssignal als Überschreiten eines Flussgrenzwerts $Q_m$ erfasst. Zu diesem Zeitpunkt ($t_1$) wird der Arbeitsdruck auf einen niedrigeren Saugdruck $p_1$ geregelt, bis die Pumpfluidkammer 7 gefüllt ist (Zeitpunkt $t_2$). Dann wird auf einen Entleerungsdruck $p_3$ gewechselt. Der so eingestellte Gegenpuls hat u. a. die Eigenschaft, dass Ansaugereignisse vermieden werden.

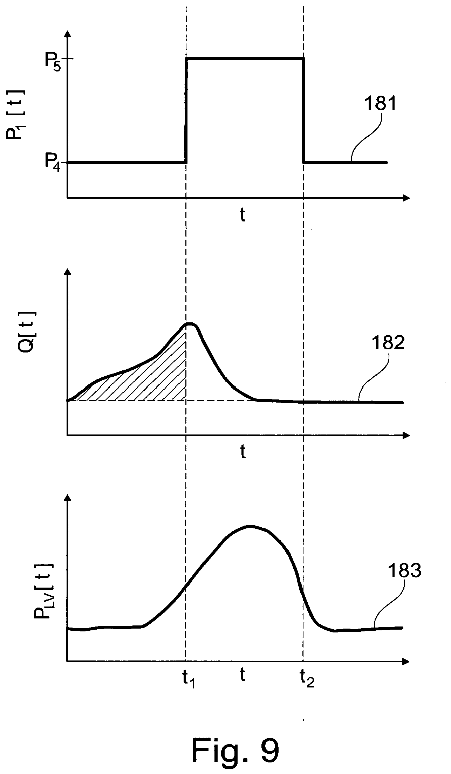

In einer Abwandlung des ersten Beispiels wird ein verzögerter Gleichpuls eingestellt. Diese Art der Synchronisation ist in Fig. 9 illustriert, wobei wieder Zeitverläufe des Arbeitsdrucks $p_A(t)$ (Kurve 181), des ersten Kanülenflusssignals Q(t) (Kurve 182) und des Ventrikeldrucks $p_{LV}(t)$ im linken Ventrikel (Kurve 183) gezeigt sind. Hierbei wird der Saugdruck $p_4$ in der Füllphase so eingestellt, dass erst der Beginn der Systole ($t_1$) die Membranfluidpumpe 2 vollständig füllt, was anhand des Integrals (schraffierter Bereich) des Einlassflusses erkannt werden kann. Bei Erkennen der Füllung wird durch Wechsel auf den Arbeitsdruck p5 die Entleerungsphase begonnen. Diese Art der Synchronisation bewirkt einen hohen Ventrikel-Druckaufbau und zuverlässige Herzklappenöffnung.

In einem weiteren Beispiel wird die Synchronisation mittels eines im Vergleich mit dem vorstehend beschriebenen Verfahren langsameren Ratenvariationsverfahren erzielt. Die Steuereinheit 4 ist, wie oben bereits beschrieben, dazu eingerichtet, eine Periodendauer des pulsatilen Fluidflusses über mehrere Pumpzyklen zu variieren,

eine durch das Variieren der Periodendauer verursachte Änderung eines pro Pumpzyklus bestimmten Maximalwertes und/oder Minimalwertes und/oder Mittelwertes und/oder Momentanwertes des ersten und/oder zweiten Kanülenflusssignals zu erfassen und

die Zeitvorgabe so zu bestimmen, dass der pro Pumpzyklus bestimmte Maximalwert und/oder Minimalwert und/oder Mittelwert und/oder Momentanwert des ersten Kanülenflusssignals maximiert oder minimiert wird.

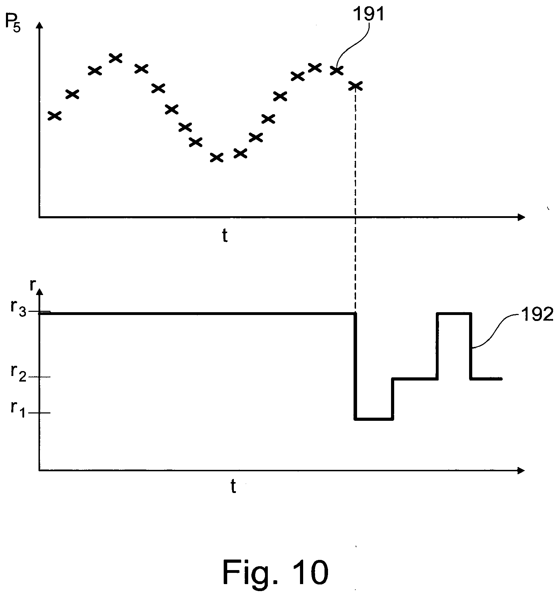

Fig. 10 zeigt ein Beispiel des Ratenvariationsverfahrens anhand von Zeitverläufen des pro Pumpzyklus bestimmten Maximalwertes des ersten Kanülenflusssignals (Spitzenfluss $p_s$, Kurve 191) und der Pumprate r (Kurve 192). Die Membranfluidpumpe 2 wird zunächst mit einer Rate $r_3$ oberhalb der Herzrate betrieben. Der Spitzenfluss im Fluideinlass 10 schwankt periodisch. Während der Abnahme des Spitzenflusses wird auf eine Rate $r_1$ unterhalb der Herzrate gewechselt, um zum gemessenen Maximum von $p_s$ zurückzukehren. Danach wird der Antrieb auf die geschätzte Herzrate eingestellt. Erneute Abweichungen vom Maximum können durch Umschalten auf $r_3$ oder $r_1$ korrigiert werden. Zugleich kann bei nötigen Korrekturen wie der dargestellten kurzfristigen Erhöhung auf $r_3$ die Herzratenschätzung aktualisiert werden.

Im Synchronisationsbetrieb ist es nicht möglich, bestimmte Parameter, etwa die Pumprate, anzupassen, ohne die Synchronisation zu stören. Dies kann jedoch vermieden werden: die Steuereinheit 4 kann dazu eingerichtet sein, den mittleren Blutfluss und/oder die relative Phasenlage durch ein Auslassen von einem oder mehreren halben oder ganzen Pumpzyklen einzustellen (wobei ein halber Pumpzyklus eine Füllungsphase oder eine Entleerungsphase sein kann).

Ein abschließendes Beispiel für die Regelung der Membranfluidpumpe 2 mittels der Steuereinheit 4 ist besonders für Failing-Fontan-Patienten geeignet, hat aber auch für andere Patienten Vorteile.

Dabei umfasst der hämodynamische Parametersatz einen Momentanfüllstand der Membranfluidpumpe, bestimmt basierend auf einem Integral des ersten Kanülenflusssignals, und die Steuereinheit 4 ist dazu eingerichtet, den Momentanfüllstand mit einem Momentansollfüllstand der Membranfluidpumpe zu vergleichen und, wenn der Momentanfüllstand den Momentansollfüllstand um mindestens einen vorgegebenen Wert unterschreitet, den Arbeitsdruck so zu regeln, dass die Pumprate des pulsatilen Fluidflusses und/oder ein Saugdruck, mit dem die Membranfluidpumpe gefüllt wird, verringert wird. Der Momentansollfüllstand ist hierbei, wie bereits beschrieben, eine zeitlich veränderliche Funktion, die einen Sollfüllstand der Membranfluidpumpe 2 zu jedem Zeitpunkt vorgibt.

Durch diese Steuerung passt sich der mittlere Blutfluss dem venösen Rückstrom an (vgl. Frank-Starling-Mechanismus) und erhält einen akzeptablen physiologischen Druck in der Hohlvene (bei Verwendung des Pumpensystems 1 als RVAD) bzw. der Pulmonalvene (bei Verwendung des Pumpensystems 1 als LVAD) aufrecht.

Bei Failing-Fontan-Patienten sollen hierbei subpulmonare Drücke zwischen 10 mmHg und 15 mmHg gehalten oder im Laufe der Therapie weiter verringert werden, was einerseits Organproblemen, andererseits einem Kollabieren der Hohlvenen vorbeugt.

Das vorgeschlagene Verfahren zum Betreiben des Pumpensystems 1 ist bereits in verschiedenen Beispielen in der Beschreibung des Pumpensystems 1 und der Steuereinheit 4 vorgestellt worden.

Das Verfahren umfasst wenigstens die Schritte:

Erfassen des ersten Kanülenflusssignals und des Arbeitsdrucksignals,

Bestimmen des Zeitversatzes und/oder des Entleerungszeitversatzes für den ersten Pumpzyklus und den ersten Herzzyklus,

Bestimmen des hämodynamischen Parametersatzes basierend auf dem Zeitversatz und/oder dem ersten Kanülenflusssignal und/oder dem Arbeitsdrucksignal,

Bestimmen der Zeitvorgabe basierend auf der Regelvorgabe derart, dass die Regelvorgabe erreicht wird.

Liste der Bezugszeichen

1 Pumpensystem,
2 Membranfluidpumpe,
3 Arbeitsdruckquelle,
4 Steuereinheit,
5 Gehäuse,
6 Membran,
7 Pumpfluidkammer,
8 Arbeitsfluidkammer,
9 Einlasskanüle,
10 Fluideinlass,
11 Auslasskanüle,
12 Fluidauslass,
13 Einlassventil,
14 Auslassventil,
15 Druckleitung,
16 Arbeitsraum,
17 Elektromotor,
18 Arbeitskolben,
19 Steuerleitung,
20 Arbeitsdrucksensor,
21 Erster Flusssensor,
22 Zweiter Flusssensor,
23 EKG-Sensoren,
24 Erster Kanülendrucksensor,

25 Zweiter Kanülendrucksensor,
26 Anzeigeeinheit,
27 Beschleunigungssensor
91, 92, 93, 101, 102, 103, 111, 112, 113, 121, 122, 131, 132, 141, 142, 143, 151,152,153, 161, 162, 163, 171, 172, 172, 181, 182, 183, 191, 192 Kurven.

**Patentansprüche**

1. Pumpensystem (1), umfassend

   eine Membranfluidpumpe (2), die mittels einer Einlasskanüle (9) und einer Auslasskanüle (11) mit einem Herzen und/oder mindestens einem Blutgefäß fluidisch verbindbar und zum Erzeugen eines pulsatilen Fluidflusses zur Unterstützung einer Herzaktivität des Herzens eingerichtet ist,
   eine Arbeitsdruckquelle (3), die mittels einer Druckleitung (15) mit der Membranfluidpumpe (2) verbunden und zum Bereitstellen eines Arbeitsdrucks zum Antreiben der Membranfluidpumpe (2) eingerichtet ist,
   eine Steuereinheit (4), eingerichtet zum Regeln des Arbeitsdrucks,
   einen ersten Flusssensor (21), eingerichtet zum Erfassen eines ersten Kanülenflusssignals, das einem Einlassfluss in der Einlasskanüle (9) oder einem Auslassfluss in der Auslasskanüle (11) entspricht,
   einen Arbeitsdrucksensor (20), eingerichtet zum Erfassen eines Arbeitsdrucksignals, das dem Arbeitsdruck in der Druckleitung (15) entspricht,
   wobei der pulsatile Fluidfluss mehrere aufeinanderfolgende Pumpzyklen umfasst, die Herzaktivität mehrere aufeinanderfolgende Herzzyklen umfasst und jeder der Pumpzyklen eine Füllungsphase und eine Entleerungsphase umfasst,

   **gekennzeichnet dadurch, dass** die Steuereinheit (4) ferner dazu eingerichtet ist,

   einen Zeitversatz zwischen einem ersten Zeitpunkt, der während eines ersten Pumpzyklus auftritt, und einem zweiten Zeitpunkt, der während eines ersten Herzzyklus auftritt, zu bestimmen,
   basierend auf dem Zeitversatz und/oder dem ersten Kanülenflusssignal und/oder dem Arbeitsdrucksignal einen hämodynamischen Parametersatz, umfassend einen oder mehrere hämodynamische Parameter, zu bestimmen und
   basierend auf einer Regelvorgabe als Vorgabe für den hämodynamischen Parametersatz und/oder für den Zeitversatz eine Zeitvorgabe als Vorgabe für einen Beginn und/oder eine Dauer der Füllungsphase und/oder der Entleerungsphase des ersten Pumpzyklus und/oder mindestens eines zweiten Pumpzyklus, der zeitlich nach dem ersten Pumpzyklus auftritt, derart zu bestimmen, dass die Regelvorgabe erreicht wird.

2. Pumpensystem (1) nach Anspruch 1, wobei die Steuereinheit (4) dazu eingerichtet ist, den Arbeitsdruck basierend auf der Zeitvorgabe so zu regeln, dass die Regelvorgabe erreicht wird.

3. Pumpensystem (1) nach einem der vorhergehenden Ansprüche, ferner aufweisend eine Anzeigeeinheit (26), wobei die Anzeigeeinheit (26) zum Anzeigen einer basierend auf der Zeitvorgabe bestimmten vorgeschlagenen Einstellungsänderung und/oder zum Anzeigen mindestens eines hämodynamischen Parameters des hämodynamischen Parametersatzes eingerichtet ist.

4. Pumpensystem (1) nach einem der vorhergehenden Ansprüche, wobei der hämodynamische Parametersatz eine Pumprate des Herzens und/oder eine Vorlast des Herzens und/oder einen enddiastolischen Druck und/oder ein enddiastolisches Volumen und/oder eine Nachlast des Herzens und/oder eine Kontraktilität des Herzens und/oder einen Ventrikeldruck und/oder ein Ventrikelfüllvolumen und/oder einen Vorhofdruck und/oder ein Vorhoffüllvolumen und/oder ein Pumpenfüllvolumen und/oder einen Arteriendruck und/oder einen Venendruck und/oder einen mittleren Blutfluss, bestimmt als über mehrere Pumpzyklen gemittelter mittlerer Blutfluss durch die Membranfluidpumpe (2), und/oder einen einer relativen Lage der Membranfluidpumpe (2) in Bezug auf das Herz entsprechenden hydrostatischen Druck umfasst.

5. Pumpensystem (1) nach einem der vorhergehenden Ansprüche, wobei die Steuereinheit (4) dazu eingerichtet ist, den Arbeitsdruck so zu regeln, dass die Membranfluidpumpe (2) in der Füllungsphase jedes Pumpzyklus vollständig gefüllt und/oder in der Entleerungsphase jedes Pumpzyklus vollständig entleert wird.

**6.** Pumpensystem (1) nach einem der vorhergehenden Ansprüche,
wobei die Zeitvorgabe eine Vorgabe für den Beginn und/oder die Dauer der Füllungsphase und/oder der Entleerungsphase des ersten und/oder zweiten Pumpzyklus ist,
wobei der hämodynamische Parametersatz einen mittleren Blutfluss, bestimmt als über mehrere Pumpzyklen gemittelter mittlerer Blutfluss durch die Membranfluidpumpe (2), umfasst,
wobei die Regelvorgabe eine Vorgabe für den hämodynamischen Parametersatz ist und
wobei die Steuereinheit (4) weiterhin dazu eingerichtet ist, als Regelvorgabe ein Erhöhen, Absenken oder Konstanthalten des mittleren Blutflusses basierend auf einem Zeitverlauf des hämodynamischen Parametersatzes zu bestimmen.

**7.** Pumpensystem (1) nach Anspruch 6, weiterhin aufweisend einen Beschleunigungssensor (27), eingerichtet zum Erfassen eines einer Patientenaktivität entsprechenden Beschleunigungssignals, wobei die Steuereinheit (4) dazu eingerichtet ist, die Regelvorgabe basierend auf dem Beschleunigungssignal zu bestimmen, insbesondere so, dass bei einem Ansteigen der Patientenaktivität der mittlere Blutfluss erhöht wird.

**8.** Pumpensystem (1) nach Anspruch 6 oder 7, wobei der hämodynamische Parametersatz neben einer Kontraktilität des Herzens eine Vorlast des Herzens und/oder eine Nachlast des Herzens umfasst und die Steuereinheit (4) dazu eingerichtet ist,
als Regelvorgabe ein Absenken des mittleren Blutflusses zu bestimmen, wenn der Zeitverlauf des hämodynamischen Parametersatzes ein Ansteigen der Kontraktilität ohne Ansteigen der Vorlast und/oder ohne Ansteigen der Nachlast umfasst und/oder wenn der Zeitverlauf des hämodynamischen Parametersatzes ein Absinken der Vorlast ohne Absinken der Kontraktilität umfasst, und/oder
als Regelvorgabe ein Konstanthalten des mittleren Blutflusses zu bestimmen, wenn der Zeitverlauf des hämodynamischen Parametersatzes ein Ansteigen der Kontraktilität und ein Ansteigen der Vorlast und/oder der Nachlast umfasst, und/oder
als Regelvorgabe ein Erhöhen des mittleren Blutflusses zu bestimmen, wenn der Zeitverlauf des hämodynamischen Parametersatzes ein Ansteigen der Vorlast ohne Ansteigen der Kontraktilität umfasst und/oder wenn der Zeitverlauf des hämodynamischen Parametersatzes ein Ansteigen der Nachlast ohne Absinken der Vorlast und ohne Ansteigen des Ventrikelhöchstdrucks umfasst und/oder wenn der Zeitverlauf des hämodynamischen Parametersatzes ein Absinken der Kontraktilität ohne Absinken der Vorlast und/oder ohne Absinken der Nachlast umfasst.

**9.** Pumpensystem (1) nach einem der vorhergehenden Ansprüche, wobei die Steuereinheit (4) dazu eingerichtet ist, den Zeitversatz basierend auf dem ersten Kanülenflusssignal zu bestimmen.

**10.** Pumpensystem (1) nach Anspruch 9, wobei die Steuereinheit (4) dazu eingerichtet ist, den Zeitversatz gemäß der Gleichung

$$T = \frac{\tau}{2\pi} \arg\left(\left[\sqrt{\frac{1}{t_1 - t_0} \int_{t_0}^{t_1} Q(t)^2 \, dt} + i \int_{t_S}^{t_E} \left(\frac{Q(t) - \langle Q \rangle}{\sigma_Q}\right)^3 \, dt\right] e^{i\alpha}\right)$$

zu bestimmen, wobei T der Zeitversatz, $\tau$ eine Periodendauer des pulsatilen Fluidflusses, $Q(t)$ ein Zeitverlauf des ersten Kanülenflusssignals, t ein Zeitparameter, $t_S$ der Zeitpunkt, zu dem $Q(t)$ zum letzten Mal vor dem Erreichen eines Maximalflusses $Q_{max}$ weniger als 20 % von $Q_{max}$ beträgt, $t_E$ der Zeitpunkt, zu dem $Q(t)$ zum letzten Mal seit Erreichen von $Q_{max}$ weniger als 20 % von $Q_{max}$ beträgt, $\langle Q \rangle$ der Mittelwert von $Q(t)$ im Zeitintervall von $t_S$ bis $t_E$, $\sigma_Q$ die Standardabweichung von $Q(t)$ im Zeitintervall von $t_S$ bis $t_E$, i die imaginäre Einheit, $\alpha$ ein vorgegebener Winkeloffset ist.

**11.** Pumpensystem (1) nach einem der vorhergehenden Ansprüche, weiterhin umfassend
einen EKG-Sensor (23), eingerichtet zum Erfassen eines EKG-Signals, das einer elektrischen Aktivität des Herzens entspricht,
und/oder einen ersten Kanülendrucksensor (24), eingerichtet zum Erfassen eines ersten Kanülendrucksignals, das einem Einlassdruck in der Einlasskanüle (9) oder einem Auslassdruck in der Auslasskanüle (11) entspricht,
wobei die Steuereinheit (4) dazu eingerichtet ist, den Zeitversatz und/oder den hämodynamischen Parametersatz basierend auf dem EKG-Signal und/oder dem ersten Kanülendrucksignal zu bestimmen.

**12.** Pumpensystem (1) nach einem der vorhergehenden Ansprüche, wobei der pulsatile Fluidfluss eine Messphase umfasst und wobei die Steuereinheit (4) dazu eingerichtet ist, für die Dauer der Messphase den Arbeitsdruck auf einen geringen Arbeitsdruckwert so einzustellen, dass eine hohe Empfindlichkeit des ersten Kanülenflusssignals für durch den ersten Herzzyklus verursachte Druckänderungen erreicht wird.

**13.** Pumpensystem (1) nach einem der Ansprüche 2 bis 12,
wobei die Zeitvorgabe eine Vorgabe für den Beginn und/oder die Dauer der Füllungsphase und/oder der Entleerungsphase des ersten und/oder zweiten Pumpzyklus ist
und wobei die Regelvorgabe eine Vorgabe für den Zeitversatz derart ist, dass eine vorgegebene relative Phasenlage des pulsatilen Fluidflusses in Bezug auf die Herzaktivität erreicht wird, insbesondere ein Gleichpuls oder ein Gegenpuls oder ein um ein vorgegebenes Verzögerungsintervall verzögerter Puls.

**14.** Pumpensystem (1) nach einem der Ansprüche 2 bis 13, wobei die Steuereinheit (4) dazu eingerichtet ist,
eine Periodendauer des pulsatilen Fluidflusses über mehrere Pumpzyklen zu variieren,
eine durch das Variieren der Periodendauer verursachte Änderung eines pro Pumpzyklus bestimmten Maximalwertes und/oder Minimalwertes und/oder Mittelwertes und/oder Momentanwertes des ersten Kanülenflusssignals zu erfassen und
die Zeitvorgabe so zu bestimmen, dass der pro Pumpzyklus bestimmte Maximalwert und/oder Minimalwert und/oder Mittelwert und/oder Momentanwert des ersten Kanülenflusssignals maximiert oder minimiert wird.

**15.** Pumpensystem (1) nach einem der vorhergehenden Ansprüche,
wobei die Regelvorgabe eine Vorgabe für den Zeitversatz ist und
wobei die Steuereinheit (4) dazu eingerichtet ist, bei einem Erfassen einer Abweichung des durch die Steuereinheit (4) bestimmten Zeitversatzes von der Regelvorgabe die Periodendauer des pulsatilen Fluidflusses über mehrere Pumpzyklen in eine erste Richtung zu ändern, bis ein Vorzeichenwechsel einer Ableitung eines pro Pumpzyklus bestimmten Maximalwertes und/oder Minimalwertes und/oder Mittelwertes und/oder Momentanwertes und/oder eines sonstigen Zeitverlaufsmerkmals des ersten Kanülenflusssignals erfasst wird,
und ferner dazu eingerichtet ist, beim Erfassen des Vorzeichenwechsels die Periodendauer des pulsatilen Fluidflusses in die der ersten Richtung entgegengesetzte Richtung zu ändern.

**16.** Pumpensystem (1) nach einem der vorhergehenden Ansprüche, wobei die Steuereinheit (4) dazu eingerichtet ist, basierend auf einem Zeitverlauf des ersten Kanülenflusssignals einen Druck und/oder eine Druckänderung und/oder eine Druckänderungsrate in einem mit der Membranfluidpumpe (2) verbundenen Ventrikel und/oder Vorhof und/oder Blutgefäß zu bestimmen.

**17.** Pumpensystem (1) nach Anspruch 16, wobei die Steuereinheit (4) dazu eingerichtet ist, den Zeitversatz durch Variieren der Zeitvorgabe über mehrere Pumpzyklen so zu variieren, dass der Druck und/oder die Druckänderung und/oder die Druckänderungsrate in dem mit der Membranfluidpumpe (2) verbundenen Ventrikel und/oder Vorhof und/oder Blutgefäß basierend auf einer durch das Variieren des Zeitversatzes verursachten Änderung des Zeitverlaufs des ersten Kanülenflusssignals bestimmbar ist.

**18.** Pumpensystem (1) nach einem der vorhergehenden Ansprüche,
wobei der hämodynamische Parametersatz einen Momentanfüllstand der Membranfluidpumpe (2), bestimmt basierend auf einem Integral des ersten Kanülenflusssignals, umfasst und
wobei die Steuereinheit (4) dazu eingerichtet ist, den Momentanfüllstand mit einem zeitlich veränderlichen Momentansollfüllstand der Membranfluidpumpe (2) zu vergleichen und, wenn der Momentanfüllstand den Momentansollfüllstand um mindestens einen vorgegebenen Wert unterschreitet, den Arbeitsdruck so zu regeln, dass eine Pumprate des pulsatilen Fluidflusses und/oder ein Saugdruck, mit dem die Membranfluidpumpe gefüllt wird, verringert wird.

**19.** Pumpensystem (1) nach Anspruch 18, wobei die Steuereinheit (4) ferner dazu eingerichtet ist, die Pumprate des pulsatilen Fluidflusses über mehrere Pumpzyklen zu erhöhen, bis der Momentanfüllstand den Momentansollfüllstand um mindestens den vorgegebenen Wert unterschreitet, und die Pumprate des pulsatilen Fluidflusses anschließend zu verringern.

**20.** Steuereinheit (4) für ein Pumpensystem (1) nach einem der Ansprüche 1 bis 19, wobei die Steuereinheit (4) zum Regeln des Arbeitsdrucks und ferner dazu eingerichtet ist,
den Zeitversatz zu bestimmen,
basierend auf dem Zeitversatz sowie basierend auf dem ersten Kanülenflusssignal und/oder dem Arbeitsdrucksignal den hämodynamischen Parametersatz zu bestimmen und
basierend auf der Regelvorgabe den Zeitversatz derart zu bestimmen, dass die Regelvorgabe erreicht wird.

21. Verfahren zum Betreiben eines Pumpensystems (1) nach einem der Ansprüche 1 bis 19, umfassend die Schritte:

Erfassen des ersten Kanülenflusssignals und des Arbeitsdrucksignals,
Bestimmen des Zeitversatzes für den ersten Pumpzyklus und den ersten Herzzyklus,
Bestimmen des hämodynamischen Parametersatzes basierend auf dem Zeitversatz sowie basierend auf dem ersten Kanülenflusssignal und/oder dem Arbeitsdrucksignal,
Bestimmen der Zeitvorgabe basierend auf der Regelvorgabe derart, dass die Regelvorgabe erreicht wird.

Fig. 1

Fig. 2

Fig. 3

Fig. 4

Fig. 5

Fig. 6a

Fig. 6b

Fig. 6c

Fig. 7

Fig. 8

Fig. 9

Fig. 10

Europäisches
Patentamt

European
Patent Office

Office européen
des brevets

**EUROPÄISCHER RECHERCHENBERICHT**

Nummer der Anmeldung

EP 19 20 8405

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | KITAMURA T ET AL: "INDIRECT MEASUREMENT TECHNIQUE FOR A PORTABLE ARTIFICIAL HEART DRIVE SYSTEM", ISA TRANSACTIONS, INSTRUMENT SOCIETY OF AMERICA. PITTSBURGH, US, Bd. 25, Nr. 4, 1. Januar 1986 (1986-01-01), Seiten 7-11, XP000024226, ISSN: 0019-0578 * das ganze Dokument * ----- | 1-15 | INV. A61M1/10 A61M1/12 |
| A | US 7 572 217 B1 (KOENIG STEVEN C [US] ET AL) 11. August 2009 (2009-08-11) * Spalte 5, Zeile 65 - Spalte 8, Zeile 30 * * Spalte 9, Zeile 49 - Spalte 11, Zeile 15 * * Abbildungen 1-4 * ----- | 1-15 | |
| A | DE 26 58 104 A1 (THOMA HERWIG DIPL ING DR) 1. Dezember 1977 (1977-12-01) * Seite 6, Absatz 3 - Seite 10, letzter Absatz * * Abbildungen 1-4 * ----- | 1-15 | RECHERCHIERTE SACHGEBIETE (IPC) A61M |
| A | US 3 457 909 A (LAIRD JOHN D) 29. Juli 1969 (1969-07-29) * Spalte 3, Zeile 5 - Spalte 5, Zeile 57 * * Abbildung 1 * ----- | 1-15 | |

Der vorliegende Recherchenbericht wurde für alle Patentansprüche erstellt

| Recherchenort | Abschlußdatum der Recherche | Prüfer |
|---|---|---|
| Den Haag | 15. September 2020 | Kempeneers, Johanna |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument

........................................................................
& : Mitglied der gleichen Patentfamilie, übereinstimmendes
Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 19 20 8405

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

15-09-2020

| Im Recherchenbericht angeführtes Patentdokument | Datum der Veröffentlichung | Mitglied(er) der Patentfamilie | Datum der Veröffentlichung |
|---|---|---|---|
| US 7572217 B1 | 11-08-2009 | KEINE | |
| DE 2658104 A1 | 01-12-1977 | AT 352858 B<br>DE 2658104 A1 | 10-10-1979<br>01-12-1977 |
| US 3457909 A | 29-07-1969 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3536955 A1